(19) Europäisches Patentamt
European Patent Office
Office européen des brevets

(11) Veröffentlichungsnummer : **0 025 192**
**B1**

# (12) EUROPÄISCHE PATENTSCHRIFT

(45) Veröffentlichungstag der Patentschrift : **11.05.83**

(21) Anmeldenummer : **80105126.9**

(22) Anmeldetag : **29.08.80**

(51) Int. Cl.$^3$ : **C 07 D303/48**, C 07 C 69/65, C 07 C 69/618, C 07 C 69/734, C 07 C 57/60, A 61 K 31/325, A 61 K 31/19, A 61 K 31/215// C07C57/76, C07C33/46, C07C25/02, C07C43/225, C07C59/64, C07C57/58, C07C21/24, C07C33/20, C07C143/68, C07C69/612

(54) **Substituierte Oxirancarbonsäuren, Verfahren zu ihrer Herstellung, ihre Verwendung und sie enthaltende Arzneimittel.**

(30) Priorität : **07.09.79 CH 8068/79**

(43) Veröffentlichungstag der Anmeldung : **18.03.81 Patentblatt 81/11**

(45) Bekanntmachung des Hinweises auf die Patenterteilung : **11.05.83 Patentblatt 83/19**

(84) Benannte Vertragsstaaten :
**AT BE CH DE FR GB IT LI LU NL SE**

(56) Entgegenhaltungen :
**GB A 1 531 078**
**US A 4 132 719**
**US A 4 132 720**
**US A 4 196 300**

**CHEMICAL ABSTRACTS, Columbus, Ohio, USA 8th Collective Index, Subjects 1973, Seite 13995s, Spalte 2.**

(73) Patentinhaber : **Byk Gulden Lomberg Chemische Fabrik GmbH**
**Byk-Gulden-Strasse 2**
**D-7750 Konstanz (DE)**

(72) Erfinder : **Amschler, Hermann, Dr.**
**Hohenhewenstrasse 19**
**D-7760 Radolfzell (DE)**
Erfinder : **Eistetter, Klaus, Dr.**
**Säntisblick 7**
**D-7750 Konstanz 19 (DE)**
Erfinder : **Ludwig, Gerhard, Dr.**
**Meisenweg 6**
**D-7750 Konstanz 16 (DE)**
Erfinder : **Rapp, Erich, Dr.**
**Pfarrer-Braun-Strasse 6**
**D-7760 Radolfzell 17 (DE)**
Erfinder : **Wolf, Horst, Dr.**
**Brandesstrasse 29**
**D-7750 Konstanz (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Substituierte Oxirancarbonsäuren, Verfahren zu ihrer Herstellung, ihre Verwendung und sie enthaltende Arzneimittel

Die Erfindung betrifft substituierte Oxirancarbonsäuren, Verfahren zu ihrer Herstellung, ihre Verwendung und sie enthaltende Arzneimittel.

Im Rahmen einer Untersuchung über die Fähigkeit von substituierten Dreiringverbindungen, als Substrat oder Inhibitor für Meerschweinchen-Lebermicrosomen-Epoxidhydrase zu dienen, wurden u. a. Phenyloxirancarbonsäureester, z. B. 2-Phenyl-oxiran-2-carbonsäureethylester, eingesetzt [F. Oesch et al., Biochem. 10(1971) N°. 26, 4858-66]. Im Verlauf der Strukturaufklärung von Isamsäure [P. de Mayo und J. J. Ryan, Can. J. Chem. 45(1967) 2177-2190] wurde Methyl-2-(o-acetaminobenzyl)-glycidat durch Reaktion von Methyl-N-acetylisatinat mit überschüssigem Diazomethan erhalten. Bestimmte substituierte Oxirancarbonsäuren wurden nun als pharmazeutische Wirkstoffe mit einer spezifischen Wirkung erfunden.

Gegenstand der Erfindung sind substituierte Oxirancarbonsäuren der allgemeinen Formel I

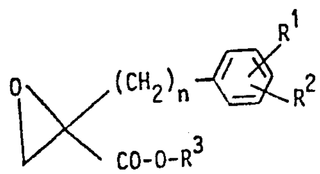

(I)

worin

$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,

$R^2$ eine der Bedeutungen von $R^1$ hat,

$R^3$ ein Wasserstoffatom oder eine $C_1$-$C_4$ Alkylgruppe und

n eine ganze Zahl von 1 bis 8 bedeuten,

sowie die Salze der Carbonsäuren.

Als $C_1$-$C_4$ Alkylgruppen kommen geradkettige oder verzweigte Alkylreste in Betracht. Geradkettige Alkylreste sind beispielsweise der Methyl-, Ethyl-, n-Propyl- und n-Butylrest, von denen die mit 1 und 2 Kohlenstoffatomen bevorzugt sind. Verzweigte Alkylreste sind beispielsweise der Isopropyl-, Isobutyl- und sek.-Butylrest, von denen der mit 3 Kohlenstoffatomen bevorzugt ist. Als $C_1$-$C_4$ Alkoxygruppen kommen sowohl geradkettige als auch verzweigte Alkoxygruppen in Frage. Die Methoxygruppe ist bevorzugt. Halogenatome sind Fluor-, Chlor- und Bromatome, von denen Fluor, insbesondere Chlor bevorzugt sind.

Die Substituenten $R^1$ und $R^2$ befinden sich bevorzugt in meta- oder para-Position.

Als Salze kommen Salze mit anorganischen und organischen Basen in Betracht. Pharmakologisch nicht verträgliche Salze werden nach an sich bekannten Methoden in pharmakologisch, d. h. biologisch, verträgliche Salze übergeführt, die unter den erfindungsgemäßen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker oder basische Aminosäuren zu Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, N-Niedrigalkylpiperazin (z. B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin und Chinolin genannt.

Eine Ausgestaltung der Erfindung sind substituierte Oxirancarbonsäuren der allgemeinen Formel I*

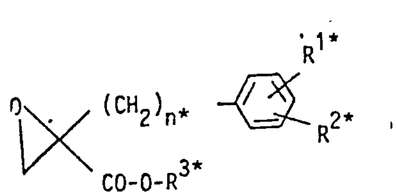

(I*)

worin

$R^{1*}$ und $R^{2*}$ meta- oder para-ständig sind und

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom,

$R^{3*}$ ein Wasserstoffatom oder eine $C_1$-$C_4$ Alkylgruppe und
$n^*$ eine ganze Zahl von 3 bis 7 bedeuten,
sowie die Salze der Carbonsäuren.

Eine bevorzugte Ausgestaltung der Erfindung sind substituierte Oxirancarbonsäuren der allgemeinen Formel I**

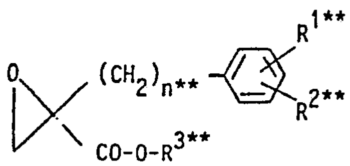

(I**)

worin
$R^{1**}$ und $R^{2**}$ meta- oder para-ständig sind und
$R^{1**}$ ein Wasserstoffatom, eine Chloratom oder eine Trifluormethylgruppe,
$R^{2**}$ ein Wasserstoffatom,
$R^{3**}$ ein Wasserstoffatom, eine Methylgruppe oder eine Ethylgruppe und
$n^{**}$ 3 oder 4 bedeuten,
sowie die pharmakologisch verträglichen Salze der Carbonsäuren mit anorganischen oder organischen Basen.

Eine besonders bevorzugte Ausgestaltung der Erfindung sind substituierte Oxirancarbonsäuren der allgemeinen Formel I***

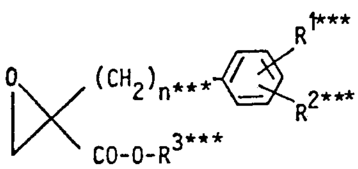

(I***)

worin
$R^{1***}$ und $R^{2***}$ meta- oder para-ständig sind und
$R^{1***}$ ein Wasserstoffatom, ein Chloratom oder eine Trifluormethylgruppe,
$R^{2***}$ ein Wasserstoffatom,
$R^{3***}$ ein Wasserstoffatom, eine Methylgruppe oder eine Ethylgruppe und
$n^{***}$ 5 bedeuten,
sowie die pharmakologisch verträglichen Salze der Carbonsäuren mit anorganischen und organischen Basen.

Als Vertreter der erfindungsgemäßen Verbindungen seien beispielsweise
2-(4-Chlorbenzyl)-oxiran-2-carbonsäureethylester,
2-(3-Chlorbenzyl)-oxiran-2-carbonsäuremethylester,
2-(4-Fluorbenzyl)-oxiran-2-carbonsäureisopropylester,
2-[2-(4-Chlorphenyl)-ethyl]-oxiran-2-carbonsäure-n-butylester,
2-[2-(4-Methoxyphenyl)-ethyl]-oxiran-2-carbonsäure-methylester,
2-[2-(3-Trifluormethylphenyl)-ethyl]-oxiran-2-carbonsäure-ethylester,
2-[3-(3-Fluorphenyl)-propyl]-oxiran-2-carbonsäure-ethylester,
2-[3-(4-Bromphenyl)-propyl]-oxiran-2-carbonsäure-methylester,
2-[3-(3-Methoxyphenyl)-propyl]-oxiran-2-carbonsäure-sek.-butylester,
2-[3-(4-n-Butoxyphenyl)-propyl]-oxiran-2-carbonsäure-ethylester,
2-[3-(3-Isopropoxyphenyl)-propyl]-oxiran-2-carbonsäure-isopropylester,
2-[4-(3-Fluorphenyl)-butyl]-oxiran-2-carbonsäure-methylester,
2-[4-(3-Trifluormethylphenyl)-butyl]-oxiran-2-carbonsäure-n-butylester,
2-[4-(3-Bromphenyl)-butyl]-oxiran-2-carbonsäure-ethylester,
2-[4-Chlorphenyl)-butyl]-oxiran-2-carbonsäure-n-propylester,
2-[4-(3,4-Dichlorphenyl)-butyl]-oxiran-2-carbonsäure-ethylester,
2-[4-(3-Chlor-4-methylphenyl)-butyl]-oxiran-2-carbonsäure-methylester,
2-[4-(4-Ethoxyphenyl)-butyl]-oxiran-2-carbonsäure-ethylester,
2-[5-(4-Methoxyphenyl)-pentyl]-oxiran-2-carbonsäure-ethylester,
2-[5-(3-Trifluormethylphenyl)-pentyl]-oxiran-2-carbonsäure-n-butylester,
2-[5-(4-Methylphenyl)-pentyl]-oxiran-2-carbonsäure-methylester,
2-[5-(3-Chlorphenyl)-pentyl]-oxiran-2-carbonsäure-isobutylester,
2-[5-(4-Methoxyphenyl)-pentyl]-oxiran-2-carbonsäure-ethylester,

2-[6-(4-Fluorphenyl)-hexyl]-oxiran-2-carbonsäure-ethylester,
2-[6-(3-Trifluormethylphenyl)-hexyl]-oxiran-2-carbonsäure-methylester,
2-[6-(3,4-Dichlorphenyl)-hexyl]-oxiran-2-carbonsäure-n-butylester,
2-[6-(4-Chlorphenyl)-hexyl]-oxiran-2-carbonsäure-isopropylester,
2-[7-(3-Fluorphenyl)-heptyl]-oxiran-2-carbonsäure-ethylester,
2-[7-(4-Trifluormethylphenyl)-heptyl]-oxiran-2-carbonsäure-ethylester,
2-[7-(3-Chlor-4-methylphenyl)-heptyl]-oxiran-2-carbonsäure-methylester,
2-[7-(3-Chlorphenyl)-heptyl]-oxiran-2-carbonsäure-n-propylester,
2-[8-(4-Fluorphenyl)-octyl]-oxiran-2-carbonsäure-ethylester,
2-[8-(3-Trifluormethylphenyl)-octyl]-oxiran-2-carbonsäure-methylester,
2-[8-(3,4-Dichlorphenyl)-octyl]-oxiran-2-carbonsäure-ethylester,
2-[8-(4-Chlorphenyl)-octyl]-oxiran-2-carbonsäure-isobutylester,

die entsprechenden Oxiran-2-carbonsäuren sowie deren Salze mit anorganischen und organischen Basen genannt.

Bevorzugte Vertreter sind

2-[3-(3-Chlorphenyl)-propyl]-oxiran-2-carbonsäureethylester,
2-[3-(4-Chlorphenyl)-propyl]-oxiran-2-carbonsäureethylester,
2-[3-(3-Trifluormethylphenyl)-propyl]-oxiran-2-carbonsäureethylester,
2-[3-(3-Trifluormethylphenyl)-propyl]-oxiran-2-carbonsäureethylester,
2-(5-Phenylpentyl)-oxiran-2-carbonsäure-ethylester,
2-[5-(4-Chlorphenyl)-pentyl]-oxiran-2-carbonsäure-ethylester,

die entsprechenden Oxiran-2-carbonsäuren sowie deren pharmakologisch verträglichen Salze.

Die substituierten Oxirancarbonsäuren der allgemeinen Formel I bzw. der Ausgestaltungen I*, I** und I*** besitzen ein Chiralitätszentrum. Die Erfindung schließt daher sowohl die Racemate und die Enantiomeren als auch deren Gemische ein.

Die erfindungsgemäßen Verbindungen weisen wertvolle pharmakologische Eigenschaften auf, die sie gewerblich verwertbar machen. Sie wirken hypoglycämisch und hypoketonämisch.

Aufgrund ihrer vorteilhaften Wirksamkeit sind die erfindungsgemäßen substituierten Oxirancarbonsäuren der allgemeinen Formel I bzw. der Ausgestaltungen I*, I** und I*** sowie die pharmakologisch verträglichen Salze zur human- und veterinärmedizinischen Behandlung und Prophylaxe von Krankheiten, die auf Störungen des Glucose- und Fettstoffwechsels beruhen, geeignet. Beispielsweise werden behandelt prädiabetische Zustände zur Verhinderung der Manifestation des Diabetes, manifester Diabetes, z. B. Erwachsenendiabetes, labiler Diabetes von Jugendlichen sowie alle krankhaften Zustände, die mit einer pathologisch erhöhten Ketonkörperproduktion einhergehen.

Gegenstand der Erfindung sind daher auch die erfindungsgemäßen Verbindungen zur Anwendung bei der Bekämpfung der vorstehend angegebenen Krankheiten.

Ein weiterer Gegenstand der Erfindung sind Arzneimittel, die eine oder mehrere der substituierten Oxirancarbonsäuren der allgemeinen Formel I

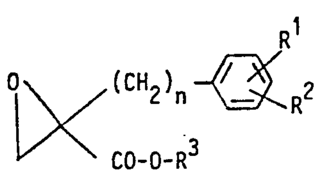

(I)

worin

$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,

$R^2$ eine der Bedeutungen von $R^1$ hat,

$R^3$ ein Wasserstoffatom oder eine $C_1$-$C_4$ Alkylgruppe und

n eine ganze Zahl von 1 bis 8 bedeuten,

und/oder die pharmakologisch verträglichen Salze der Säuren mit anorganischen oder organischen Basen enthalten.

Ausgestaltungen der Arzneimittel sind solche, die substituierte Oxirancarbonsäuren der Ausgestaltungen I*, I** oder I*** und/oder die pharmakologisch verträglichen Salze der Säuren mit anorganischen oder organischen Basen, enthalten.

Gegenstand der Erfindung ist außerdem die Verwendung der erfindungsgemässen Verbindungen zur Herstellung von Arzneimitteln zur Bekämpfung der angegebenen Krankheiten.

Die Arzneimittel werden nach an sich bekannten Verfahren hergestellt. Als Arzneimittel werden die erfindungsgemäßen Verbindungen entweder als solche oder gegebenenfalls in Kombination mit

geeigneten pharmazeutischen Trägerstoffen eingesetzt. Enthalten die pharmazeutischen Zubereitungen neben den Wirkstoffen pharmazeutische Trägerstoffe, beträgt der Wirkstoffgehalt dieser Mischungen 1 bis 95, vorzugsweise 10 bis 85, Gewichtsprozent der Gesamtmischung. Die Arzneimittel werden beispielsweise für die orale oder parenterale (intravenöse, intramusculäre) Gabe in geeigneten Dosen formuliert. Die Tagesdosis für die orale Applikation an Menschen liegt im allgemeinen zwischen 0,1 und 30, vorzugsweise 0,3 bis 15, insbesondere 0,6 bis 3 mg/kg Körpergewicht. Die Dosierung für die parenterale Behandlung liegt zwischen 0,3 bis 1 mg Wirkstoff/Körpergewicht.

Die pharmazeutischen Zubereitungen bestehen bevorzugt aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Neben den erfindungsgemäßen substituierten Oxirancarbonsäuren, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u. a.), z. B. Carbutamid, Tolbutamid, Chlorpropamid, Glibenclamid, Glibornurid, Glisoxepid, Gliquidon, Glymidin oder Hypolipidämika, wie Nicotinsäure sowie deren Derivate und Salze, enthalten.

Ein weiterer Gegenstand der Erfindung ist ein Verfahren zur Herstellung der substituierten Oxirancarbonsäuren der allgemeinen Formel I

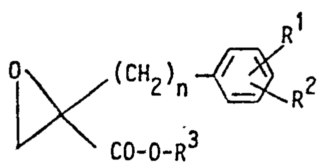

(I)

worin

$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,

$R^2$ eine der Bedeutungen von $R^1$ hat,

$R^3$ ein Wasserstoffatom oder eine $C_1$-$C_4$ Alkylgruppe und

n eine ganze Zahl von 1 bis 8 bedeuten,

sowie der Salze der Säuren, dadurch gekennzeichnet, daß man eine substituierte 2-Methylencarbonsäure der allgemeinen Formel II

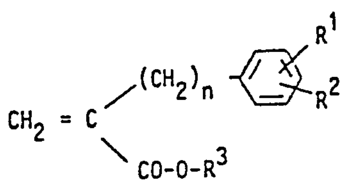

(II)

wobei

$R^1$, $R^2$, $R^3$ und n die vorstehend angegebene Bedeutung haben, oxidiert und gegebenenfalls anschließend die erhaltenen Niederalkylester verseift oder die erhaltenen Säuren in die Salze oder $C_1$-$C_4$ Alkylester überführt.

Die Oxidation der α-Methylencarbonsäuren II erfolgt unter den Bedingungen, wie sie dem Fachmann für die Oxidation von Kohlenstoff-Kohlenstoff-Doppelbindungen zu Epoxiden bekannt sind. Als Oxidationsmittel kommen beispielweise Peroxoverbindungen, wie Wasserstoffperoxid, Peressigsäure, Trifluorperessigsäure, 3,5-Dinitroperbenzoesäure, bevorzugt m-Chlorperbenzoesäure in Betracht. Die Reaktion wird zweckmäßigerweise in inerten Lösungsmitteln, z. B. aromatischen oder chlorierten Kohlenwasserstoffen, wie Benzol, Toluol, Dichlormethan, Chloroform durchgeführt. Die Reaktionstemperaturen liegen zwischen 0 °C und der Siedetemperatur des Lösungsmittels, bevorzugt zwischen 20 und 70 °C.

Die Verseifung der Niederalkylester erfolgt in an sich bekannter Weise. Sie wird beispielsweise mit einer wässerigen oder alkoholischen (z. B. ethanolischen) Alkalimetallhydroxid- (z. B. Kaliumhydroxid-) Lösung bei Raumtemperatur vorgenommen, gegebenenfalls unter Zusatz eines inerten Verdünnungsmittels, wie Dioxan oder Toluol.

Die Überführung der Säuren der allgemeinen Formel I ($R^3$ = —H) bzw. der Ausgestaltungen I*, I**

und I*** in die Salze kann durch direkte alkalische Hydrolyse der Säurederivate I ($R^3$ = $C_1$-$C_4$ Alkyl) erfolgen. Als alkalischer Reaktionspartner wird diejenige anorganische oder organische Base verwendet, deren Salz gewünscht wird. Man erhält die Salze aber auch, indem man die Säuren I ($R^3$ = —H) mit dem stöchiometrischen Äquivalent an entsprechender Base, z. B. Natriumhydroxid oder Natriumethanolat umsetzt, oder leicht lösliche Salze durch doppelte Umsetzung in schwer lösliche Salze überführt, oder beliebige Salze in pharmakologisch verträgliche Salze überführt.

Die Überführung der Oxirancarbonsäuren der allgemeinen Formel I ($R^3$ = —H) bzw. der Ausgestaltungen I*, I** und I*** in die Niederalkylester ($R^3$ = $C_1$-$C_4$ Alkyl) erfolgt in üblicher Weise. Beispielsweise werden sie mit Niederalkanolen in Gegenwart von starken Säuren, wie Schwefelsäure, p-Toluolsulfonsäure, oder von sauren Ionenaustauschern unter Bedingungen, bei denen Keine Decarboxylierung stattfindet, oder mit Dialkylsulfaten oder Alkylhalogeniden in Gegenwart von Diazabicycloundecen oder Diazabicyclononen in inerten Lösungsmitteln, wie Benzol, Toluol, Aceton, verestert.

Die Verbindungen der allgemeinen Formel I fallen normalerweise in Form von racemischen Gemischen an, die mittels bekannter Verfahren in die Enantiomeren getrennt werden. Beispielsweise wandelt man mit einem optisch aktiven Spaltungsmittel das Racemat in Diastereomere um, die anschließend durch selektive Kristallisation getrennt und in die entsprechenden optischen Isomeren übergeführt werden. Als optisch aktive Spaltungsmittel dienen z. B. optisch aktive Basen, wie 1- und d-1-Phenylethylamin, Cinchonidin oder d-Ephedrin, aus denen Salze der Säuren der allgemeinen Formel I, oder optisch aktive Alkohole, wie Borneol oder Menthol, aus denen Ester der Säuren der allgemeinen Formel I hergestellt werden. Man kann auch racemische Gemische durch Chromatographie über optisch aktive Sorptionsmittel in die optischen Isomeren zerlegen. Alternativ werden zunächst die α-Methylencarbonsäuren II mit einem optisch aktiven Spaltungsmittel umgesetzt, z. B. Borneol oder Menthol, die erhaltenen Produkte werden zu den entsprechenden Diastereomerengemischen der Oxirancarbonsäureester oxydiert, aus denen dann in üblicher Weise die optischen Isomeren der Säuren I gewonnen werden.

Zur Herstellung der substituierten Oxirancarbonsäuren der Ausgestaltungen I*, I** und I*** werden α-Methylencarbonsäuren der allgemeinen Formeln II*, II** und II***

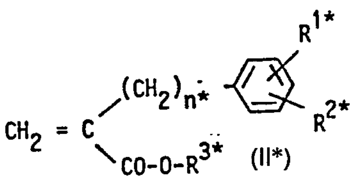
(II*)

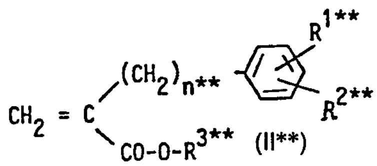
(II**)

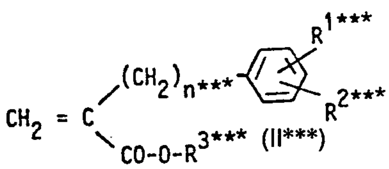
(II***)

worin

$R^{1*}$, $R^{2*}$, $R^{3*}$ und n* bzw. $R^{1**}$, $R^{2**}$, $R^{3**}$ und n** bzw. $R^{1***}$, $R^{2***}$, $R^{3***}$ und n*** die oben angegebene Bedeutung haben, eingesetzt.

Die α-Methylencarbonsäuren der allgemeinen Formeln II, II*, II** und II*** sind bekannt oder können nach an sich bekannten Methoden hergestellt werden. Sie sind wertvolle Zwischenprodukte für die Synthese der Oxirancarbonsäuren I, I*, I** und I***. Darüber hinaus besitzen die α-Methylencarbonsäuren der allgemeinen Formel II**, in der $R^{1**}$ ein Chloratom bedeutet und $R^{2**}$, $R^{3**}$ und n** die oben angegebene Bedeutung haben, und der allgemeinen Formel II***, in der $R^{1***}$, $R^{2***}$, $R^{3***}$ und n*** die oben angebene Wirkung haben, ebenfalls eine hypoglykämische Wirkung. Beispielsweise seien 5-(3-Chlorphenyl)-α-methylenvaleriansäureethylester, 7-Phenyl-α-methylenheptansäure, 7-(4-Chlorphenyl)-α-methylenheptansäure und insbesondere 5-(4-Chlorphenyl)-α-methylenvaleriansäureethylester erwähnt. Die Verbindungen der allgemeinen Formel II**, in der $R^{1**}$ ein Chloratom bedeutet und $R^{2**}$, $R^{3**}$ und n** die oben angegebene Bedeutung haben, und der allgemeinen Formel II***, in der $R^{1***}$, $R^{2***}$, $R^{3***}$ und n*** die oben angegebene Bedeutung haben, können also nicht nur als Zwischenprodukt, sondern auch als Arzneimittel Verwendung finden. Die Formulierung der Arzneimittel erfolgt in an sich bekannter Weise nach den bereits beschriebenen Methoden.

Die Herstellung der α-Methylencarbonsäuren II erfolgt beispielsweise in Analogie zu H. Stetter und H. Kuhlmann [Synthesis *1979*, 29] durch Umsetzung von Malonsäurehalbestern der allgemeinen Formel III

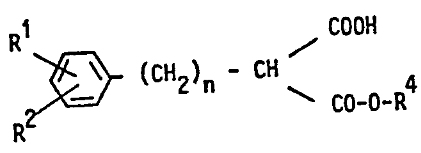
(III)

worin

$R^1$, $R^2$ und n die oben angegebene Bedeutung haben und $R^4$ eine Niederalkylgruppe bedeutet, mit Formaldehyd in Pyridin in Gegenwart von sekundären Aminen, vorzugsweise Piperidin, und gegebenenfalls anschließende Verseifung der so erhaltenen Niederalkylester.

Alternativ können die α-Methylencarbonsäuren II in Analogie zu den von Ph. E. Pfeffer et al. [J. Org. Chem. 37 (1972) 1256] und W. S. Wadsworth, jr. und W. D. Emmons [J. Am. Chem. Soc. 83 (1961) 1733] beschriebenen Methoden hergestellt werden. 5-Phenyl-α-methylenvaleriansäure und 3-Phenyl-α-methylenpropionsäure wurden von C. Mannich und E. Ganz [Ber. Dtsch. Chem. Ges. 55 (1922) 3486] beschrieben. 3-Phenyl-α-methylenpropionsäureethylester wurde von Y. Ueno et al. [Tetrahedron Letters *1978*, 3753], 4-Phenyl-α-methylenbuttersäureethylester wurde von F. Hahne und F. Zymalkowski [Arch. Pharm. 312 (1979) 472] beschrieben.

Die Herstellung der Malonsäurehalbester III erfolgt nach Methoden wie sie dem Fachmann geläufig sind, beispielsweise durch Umsetzung von Dialkylmalonaten IV mit Phenylalkylverbindungen V und partielle Hydrolyse der erhaltenen Malonsäurediester VI nach dem folgenden Schema

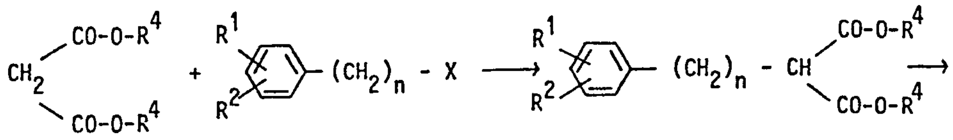 (III)

(IV) (V) (VI)

wobei

$R^1$, $R^2$, $R^4$ und n die oben angegebene Bedeutung haben und X eine Fluchtgruppe, z. B. ein Chlor- oder Bromatom, eine Mesyloxy- oder eine p-Toluolsulfonyloxygruppe, bedeutet.

Für die Herstellung der α-Methylencarbonsäuren II*, II** bzw. II*** werden entsprechende Ausgangsverbindungen III*, III** bzw. III***

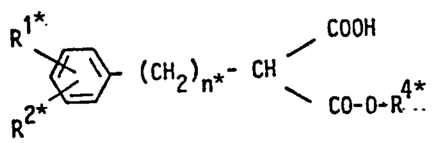 (III*)

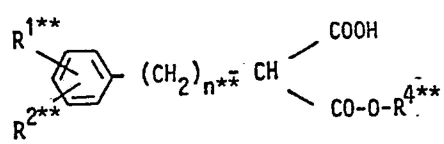 (III**)

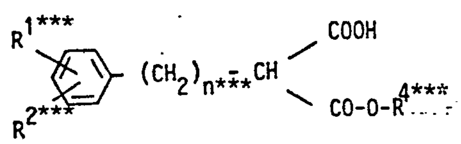 (III***)

IV*, IV** bzw. IV***

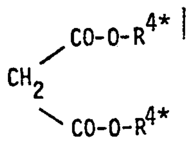 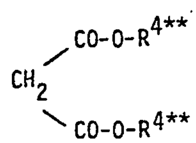 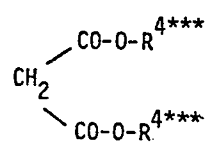

(IV*) (IV**) (IV***)

V*, V** bzw. V***

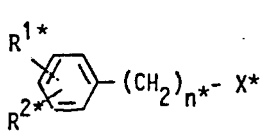 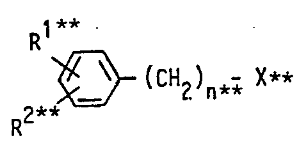 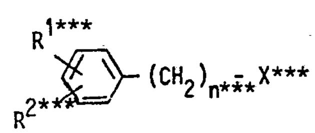

(V*) (V**) (V***)

VI*, VI** bzw. VI***

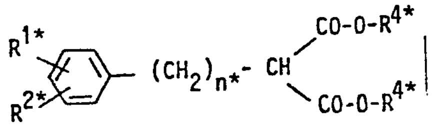

(VI*)

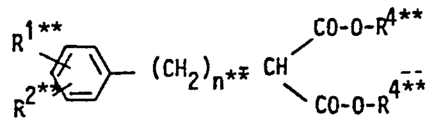

(VI**)

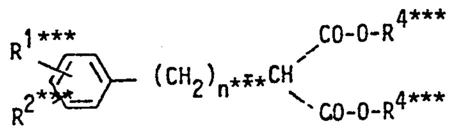

(VI***)

eingesetzt, in denen $R^{1*}$, $R^{2*}$ und n* bzw. $R^{1**}$, $R^{2**}$ und n** bzw. $R^{1***}$, $R^{2***}$ und n*** die oben angegebenen Bedeutungen haben ; $R^{4*}$ eine Niedrigalkylgruppe, $R^{4**}$ bzw. $R^{4***}$ eine Methyl- oder Ethylgruppe und X*, X** bzw. X*** ein Chlor- oder Bromatom, eine Mesyloxy- oder eine p-Toluolsulfonyloxygruppe bedeuten.

Die folgenden Beispiele dienen zur Erläuterung der Erfindung, ohne sie zu beschränken. Kp. bedeutet Siedepunkt, F. bedeutet Schmelzpunkt. Temperaturangaben erfolgen in °C.

## Beispiele

### Beispiel 1

2-(3-Phenylpropyl)-oxiran-2-carbonsäureethylester

a) 2-(3-Phenylpropyl)-oxiran-2-carbonsäureethylester

10 g 5-Phenyl-2-methylenvaleriansäureethylester und 18,3 g m-Chlorperbenzoesäure (85 %ig) werden in 180 ml Methylenchlorid 40 Stunden unter Rückfluß gekocht. Man läßt abkühlen, filtriert von der abgeschiedenen m-Chlorbenzoesäure ab, engt das Filtrat ein, nimmt den Rückstand mit 40 ml Aceton auf, gibt 20 ml einer gesättigten Natriumcarbonatlösung hinzu und rührt 1 Stunde bei 0 °C. Man verdünnt mit 100 ml Wasser, extrahiert 3 mal mit je 50 ml Methylenchlorid, engt die organische Phase ein und destilliert den Rückstand. Man erhält 8,3 g der flüssigen Titelverbindung vom Kp. 115 °C bei 0,03 Torr (4 Pa).

b) 5-Phenyl-2-methylenvaleriansäureethylester

50 g 3-Phenylpropylmalonsäuremonoethylester, 7,5 g Paraformaldehyd, 37,5 ml Pyridin und 2,5 ml Piperidin werden 5 Stunden bei 50 °C gerührt. Die Reaktionsmischung wird nach dem Abkühlen mit 350 ml Wasser versetzt und 3 mal mit je 150 ml Hexan extrahiert. Die organische Phase wird nach dem Waschen mit 1n Salzsäure, Wasser und Natriumhydrogencarbonatlösung eingeengt und destilliert. Man erhält 5-Phenyl-2-methylenvaleriansäureethylester als farblose Flüssigkeit vom Kp. 78-79 °C bei 0,02 Torr (2,66 Pa).

Alternativ wird 5-Phenyl-2-methylenvaleriansäureethylester durch Veresterung von 5-Phenyl-2-methylenvaleriansäure mit Ethanol in Gegenwart von p-Toluolsulfonsäure erhalten.

### Beispiel 2

2-(3-Phenylpropyl)-oxiran-2-carbonsäure

6,0 g 2-(3-Phenylpropyl)-oxiran-2-carbonsäureethylester, 26 ml 1n Natronlauge und 60 ml Ethanol werden 1 Stunde bei Raumtemperatur gerührt ; die Lösung wird unter Eiskühlung mit 13 ml 2n Salzsäure versetzt und im Vakuum auf ein Drittel des Volumens eingeengt. Man versetzt die Lösung mit 50 ml Wasser und extrahiert sie 3 mal mit je 50 ml Diethylether. Nach dem Trocknen der organischen Phase über Natriumsulfat und Verdampfen des Lösungsmittels erhält man 4,3 g eines zähen Öls.

Alternativ wird die Verbindung wie folgt erhalten : Eine aus 1,8 ml 85 %igem Wasserstoffperoxid, 17 g Trifluoressigsäureanhydrid und 15 ml Methylenchlorid hergestellte Lösung von Trifluorperessigsäure wird zu einer siedenden Mischung aus 9,5 g 5-Phenyl-2-methylenvaleriansäure und 28 g Dinatriumhydrogenphosphat in 50 ml Methylenchlorid getropft. Man kocht noch 30 Minuten unter Rückfluß, gibt

150 ml Eiswasser zu, stellt den pH-Wert mit Salzsäure auf 2, trennt die organische Phase ab und engt diese nach dem Trocknen über Natriumsulfat ein. Zurück bleibt die Titelverbindung als zähes Öl.

Beispiel 3

2-(4-Phenylbutyl)-oxiran-2-carbonsäureethylester

a) 2-(4-Phenylbutyl)-oxiran-2-carbonsäureethylester

Nach der in Beispiel 1a beschriebenen Arbeitsweise erhält man aus 12 g 6-Phenyl-2-methylenhexansäureethylester und 26,5 g m-Chlorperbenzoesäure in 160 ml Methylenchlorid 7,6 g der Titelverbindung vom Kp. 105 °C bei 0,005 Torr (0,66 Pa).

b) 6-Phenyl-2-methylenhexansäureethylester

Nach der in Beispiel 1b beschriebenen Arbeitsweise erhält man aus 72 g 4-Phenylbutylmalonsäuremonoethylester, 11,3 g Paraformaldehyd, 51,3 ml Pyridin und 3,4 ml Piperidin 49 g 6-Phenyl-2-methylenhexansäureethylester vom Kp. 120-125 °C bei 0,005 Torr (0,66 Pa).

c) 4-Phenylbutylmalonsäuremonoethylester

Man tropft bei Raumtemperatur eine Lösung von 17,8 g Kaliumhydroxid in 200 ml Ethanol zu 91 g 4-Phenylbutylmalonsäurediethylester in 200 ml Ethanol. Man rührt 24 Stunden, engt im Vakuum weitgehend ein, nimmt den Rückstand in 500 ml Wasser auf und extrahiert 2 mal mit je 100 ml Diethylether. Die wässerige Phase wird unter Eiskühlung mit konzentrierter Salzsäure angesäuert und 3 mal mit je 200 ml Diethylether extrahiert ; die organische Phase wird nach dem Trocknen über Natriumsulfat eingeengt. Zurück bleiben 72,8 g eines viskosen Öls.

Beispiel 4

2-[3-(2-Chlorphenyl)-propyl]-oxiran-2-carbonsäureethylester

a) 2-[3-(2-Chlorphenyl)-propyl]-oxiran-2-carbonsäureethylester

Nach der in Beispiel 1a beschriebenen Arbeitsweise erhält man aus 14 g 5-(2-Chlorphenyl)-2-methylenvaleriansäureethylester und 22,5 g m-Chlorperbenzoesäure in 170 ml Methylenchlorid 5,0 g der Titelverbindung vom Kp. 110-113 °C bei 0,005 Torr (0,66 Pa).

b) 5-(2-Chlorphenyl)-2-methylenvaleriansäureethylester

Nach der in Beispiel 1b) bechriebenen Arbeitsweise erhält man aus 71 g 3-(2-Chlorphenyl)-propylmalonsäuremonoethylester, 10,42 g Paraformaldehyd, 47 ml Pyridin und 3,1 ml Piperidin 53,3 g 5-(2-Chlorphenyl)-2-methylenvaleriansäureethylester vom Kp. 95-98° bei 0,005 Torr (0,66 Pa).

c) 3-(2-Chlorphenyl)-propylmalonsäuremonoethylester

Nach der in Beispiel 3c) beschriebenen Arbeitsweise erhält man aus 92 g 3-(2-Chlorphenyl)-propylmalonsäurediethylester und 16,8 g Kaliumhydroxid in 400 ml Ethanol 71,8 g 3-(2-Chlorphenyl)-propylmalonsäuremonoethylester als viskoses Öl.

Beispiel 5

2-[3-(3-Chlorphenyl)-propyl]-oxiran-2-carbonsäureethylester

a) 2-[3-(3-Chlorphenyl)-propyl]-oxiran-2-carbonsäureethylester

Nach der in Beispiel 1a beschriebenen Arbeitsweise erhält man aus 14 g 5-(3-Chlorphenyl)-2-methylenvaleriansäureethylester und 22,5 g m-Chlorperbenzoesäure in 170 ml Methylenchlorid 10,8 g der Titelverbindung vom Kp. 135-138 °C bei 0,005 Torr (0,66 Pa).

b) 5-(3-Chlorphenyl)-2-methylenvaleriansäureethylester

Nach der in Beispiel 1b beschriebenen Arbeitsweise erhält man aus 57 g 3-(3-Chlorphenyl)-propylmalonsäureethylester, 8,36 g Paraformaldehyd, 37,7 ml Pyridin und 2,5 ml Piperidin 35,75 g 5-(3-Chlorphenyl)-2-methylenvaleriansäureethylester vom Kp. 105-110 °C bei 0,005 Torr (0,66 Pa).

c) 3-(3-Chlorphenyl)-propylmalonsäureethylester

Nach der in Beispiel 3c) beschriebenen Arbeitsweise erhält man aus 74 g 3-(3-Chlorphenyl)-propylmalonsäurediethylester und 13,5 g Kaliumhydroxid in 200 ml Ethanol 57,7 g 3-(3-Chlorphenyl)-propylmalonsäureethylester als viskoses Öl.

d) 3-(3-Chlorphenyl)-propylmalonsäurediethylester

Man tropft bei 50 °C 91,3 g Malonsäurediethylester zu einer aus 13,11 g Natrium und 650 ml Ethanol frisch hergestellten Natriumethylatlösung. Man hält 2,5 Stunden bei dieser Temperatur und tropft anschließend 185 g p-Toluolsulfonsäure-[3-(3-chlorphenyl)-propyl]-ester zu. Nach beendeter Zugabe rührt man 6 Stunden bei 50 °C, versetzt anschließend mit 800 ml Wasser und extrahiert 3 mal mit insgesamt 1 Liter Diethylether. Die vereinigten organischen Phasen werden nach dem Trocknen über Natriumsulfat und Abdampfen des Lösungsmittels destilliert. Man erhält 74,2 g 3-(3-Chlorphenyl)-propylmalonsäurediethylester vom Kp. 145-152 °C bei 0,01 Torr (1,33 Pa).

e) p-Toluolsulfonsäure-[3-(3-chlorphenyl)-propyl]-ester

Man tropft bei 0 °C 86 ml Pyridin zu 90 g 3-(3-Chlorphenyl)-propanol-1 und 124 g p-Toluolsulfonsäurechlorid in 300 ml Chloroform. Nach beendeter Zugabe rührt man 3 Stunden bei Raumtemperatur und gießt die Lösung in eine Mischung aus 400 ml Wasser und 120 ml konzentrierter Salzsäure. Die organische Phase wird abgetrennt, 3 mal mit Wasser gewaschen, über Natriumsulfat getrocknet und im Vakuum zu einem viskosen Öl eingeengt.

Beispiel 6

2-[3-(4-Chlorphenyl)-propyl]-oxiran-2-carbonsäureethylester

a) 2-[3-(4-Chlorphenyl)-propyl]-oxiran-2-carbonsäureethylester

Nach der in Beispiel 1a) beschriebenen Arbeitsweise erhält man aus 10,0 g 5-(4-Chlorphenyl)-2-methylenvaleriansäureethylester und 20,3 g m-Chlorperbenzoesäure in 150 ml Methylenchlorid 4,3 g der Titelverbindung vom Kp. 110 °C bei 0,005 Torr (0,66 Pa).

b) 5-(4-Chlorphenyl)-2-methylenvaleriansäureethylester

Nach der in Beispiel 1b) beschriebenen Arbeitsweise erhält man aus 91,7 g 3-(4-Chlorphenyl)-propylmalonsäureethylester, 14,5 g Paraformaldehyd, 73,1 ml Pyridin und 4,8 ml Piperidin 58,8 g 5-(4-Chlorphenyl)-2-methylenvaleriansäureethylester vom Kp. 120-123 °C bei 0,05 Torr (6,65 Pa).

c) 3-(4-Chlorphenyl)-propylmalonsäureethylester

Nach der in Beispiel 3c) beschriebenen Arbeitsweise erhält man aus 125,15 g 3-(4-Chlorphenyl)-propylmalonsäurediethylester und 25,5 g Kaliumhydroxid in 500 ml Ethanol 92,2 g 3-(4-Chlorphenyl)-propylmalonsäureethylester als viskoses Öl.

d) 3-(4-Chlorphenyl)-propylmalonsäurediethylester

Nach der in Beispiel 5d) beschriebenen Arbeitsweise erhält man aus 220 g p-Toluolsulfonsäure-[3-(4-chlorphenyl)-propyl]-ester, 108,5 g Malonsäurediethylester und einer Lösung von 15,6 g Natrium in 750 ml Ethanol 105,6 g 3-(4-Chlorphenyl)-propylmalonsäurediethylester vom Kp. 145-155 °C bei 0,01 Torr (1,33 Pa).

e) p-Toluolsulfonsäure-[3-(4-chlorphenyl)-propyl]-ester

Nach der in Beispiel 5e) beschriebenen Arbeitsweise erhält man aus 150 g 3-(4-Chlorphenyl)-propanol-1, 206,6 g p-Toluolsulfonsäurechlorid und 135 ml Pyridin in 300 ml Chloroform 285 g p-Toluolsulfonsäure-[3-(4-chlorphenyl)-propyl]-ester als viskoses, gelbliches Öl.

Beispiel 7

2-[3-(4-Methoxyphenyl)-propyl]-oxiran-2-carbonsäureethylester

a) 2-[3-(4-Methoxyphenyl)-propyl]-oxiran-2-carbonsäureethylester

Nach der in Beispiel 1a) beschriebenen Arbeitsweise erhält man aus 10 g 5-(4-Methoxyphenyl)-2-methylenvaleriansäureethylester und 20 g m-Chlorperbenzoesäure in 150 ml Methylenchlorid 5,6 g der Titelverbindung vom Kp. 120-125 °C bei 0,005 Torr (0,66 Pa).

b) 5-(4-Methoxyphenyl)-2-methylenvaleriansäureethylester

Nach der in Beispiel 1b) beschriebenen Arbeitsweise erhält man aus 50 g 3-(4-Methoxyphenyl)-propylmalonsäureethylester, 33,7 ml Pyridin, 2,2 ml Piperidin und 7,4 g Paraformaldehyd 31,5 g 5-(4-Methoxyphenyl)-2-methylenvaleriansäureethylester vom Kp. 134-135 °C bei 0,05 Torr (6,65 Pa).

c) 3-(4-Methoxyphenyl)-propylmalonsäureethylester

Nach der in Beispiel 3c) beschriebenen Arbeitsweise erhält man aus 63 g 3-(4-Methoxyphenyl)-propylmalonsäurediethylester und 12,7 g Kaliumhydroxid in 250 ml Ethanol 50,3 g 3-(4-Methoxyphenyl)-propylmalonsäureethylester als viskoses Öl.

Beispiel 8

2-[3-(3-Trifluormethylphenyl)-propyl]-oxiran-2-carbonsäureethylester

a) 2-[3-(3-Trifluormethylphenyl)-propyl]-oxiran-2-carbonsäureethylester

Nach der in Beispiel 1a) beschriebenen Arbeitsweise erhält man aus 15 g 2-Methylen-5-(3-trifluormethylphenyl)-valeriansäure-ethylester und 21,27 g m-Chlorperbenzoesäure in 150 ml Methylenchlorid 8,5 g der Titelverbindung vom Kp. 110 °C bei 0,07 Torr (9,31 Pa).

b) 2-Methylen-5-(3-trifluormethylphenyl)-valeriansäureethylester

Nach der in Beispiel 1b) beschriebenen Arbeitsweise erhält man aus 35,4 g 3-(3-Trifluormethylphenyl)-propylmalonsäureethylester, 4,24 g Paraformaldehyd, 19,2 ml Pyridin und 1,27 ml Piperidin 19,53 g 2-Methylen-5-(3-trifluormethylphenyl)-valeriansäureethylester vom Kp. 98-110 °C bei 0,07 Torr (9,31 Pa).

c) 3-(3-Trifluormethylphenyl)-propylmalonsäureethylester

Nach der in Beispiel 3c) beschriebenen Arbeitsweise erhält man aus 45,3 g 3-(3-Trifluormethylphenyl)-propylmalonsäurediethylester und 7,5 g Kaliumhydroxid in 260 ml Ethanol 36,2 g 3-(3-Trifluormethylphenyl)-propylmalonsäureethylester als viskoses Öl.

d) 3-(3-Trifluormethylphenyl)-propylmalonsäurediethylester

Nach der in Beispiel 5d) beschriebenen Arbeitsweise erhält man aus 125 g p-Toluolsulfonsäure-[3-(3-trifluormethylphenyl)-propyl]-ester, 58,1 g Malonsäurediethylester und einer Lösung von 8,5 g Natrium in 400 ml Ethanol 50,6 g 3-(3-Trifluormethylphenyl)-propylmalonsäurediethylester vom Kp. 118-120 °C bei 0,07 Torr (9,31 Pa).

e) p-Toluolsulfonsäure-[3-(3-trifluormethylphenyl)-propyl]-ester

Nach der in Beispiel 5e) beschriebenen Arbeitsweise erhält man aus 71,5 g 3-(3-Trifluormethylphenyl)-propanol-1,83 g p-Toluolsulfonsäurechlorid und 54 ml Pyridin in 160 ml Chloroform 125 g p-Toluolsulfonsäure-[3-(3-trifluormethylphenyl)-propyl]-ester als gelbes Öl.

f) 3-(3-Trifluormethylphenyl)-propanol-1

Man tropft bei 0-10 °C eine Lösung von 57 g Oxiran in 120 ml Diethylether zu einer Grignard-Lösung, hergestellt aus 14,8 g Magnesium und 100 g 3-(Chlormethyl)-benzotrifluorid in 450 ml Diethylether. Man rührt 1 Stunde bei Raumtemperatur nach und versetzt anschließend mit 300 ml 10 %iger Schwefelsäure unter Eiskühlung. Man sammelt die organische Phase, extrahiert noch 2 mal mit Diethylether, trocknet die vereinigten organischen Phasen über Magnesiumsulfat und destilliert. Man erhält 76,7 g 3-(3-Trifluormethylphenyl)-propanol-1 vom Kp. 85-95 °C bei 0,02 Torr (2,66 Pa).

Beispiel 9

2-[3-(4-Methylphenyl)-propyl]-oxiran-2-carbonsäureethylester

a) 2-[3-(4-Methylphenyl)-propyl]-oxiran-2-carbonsäureethylester

Nach der in Beispiel 1a) beschriebenen Arbeitsweise erhält man aus 10 g 5-(4-Methylphenyl)-2-methylenvaleriansäureethylester und 11,8 g 3,5-Dinitroperbenzoesäure in 100 ml Methylenchlorid 6,8 g der Titelverbindung vom Kp. 110-115 °C bei 0,005 Torr (0,66 Pa).

b) 5-(4-Methylphenyl)-2-methylenvaleriansäureethylester

Nach der in Beispiel 1b) beschriebenen Arbeitsweise erhält man aus 72 g 3-(4-Methylphenyl)-propylmalonsäureethylester, 11,3 g Paraformaldehyd, 51 ml Pyridin und 3,4 ml Piperidin 46,5 g 5-(4-Methylphenyl)-2-methylenvaleriansäureethylester vom Kp. 120-128 °C bei 0,008 Torr (1,06 Pa).

c) 3-(4-Methylphenyl)-propylmalonsäureethylester

Nach der in Beispiel 3c) beschriebenen Arbeitsweise erhält man aus 90 g 3-(4-Methylphenyl)-propylmalonsäurediethylester und 17,6 g Kaliumhydroxid 73,2 g 3-(4-Methylphenyl)-propylmalonsäureethylester.

Beispiel 10

2-(5-Phenylpentyl)-oxiran-2-carbonsäureethylester

a) 2-(5-Phenylpentyl)-oxiran-2-carbonsäureethylester

Nach der in Beispiel 1a) beschriebenen Arbeitsweise erhält man aus 13 g 2-Methylen-7-phenylheptansäureethylester und 21,4 g m-Chlorperbenzoesäure in 160 ml Methylenchlorid 7,9 g der Titelverbindung vom Kp. 115-120 °C bei 0,005 Torr (0,66 Pa).

b) 2-Methylen-7-phenylheptansäureethylester

Nach der in Beispiel 1b) beschriebenen Arbeitsweise erhält man aus 44 g 5-Phenylpentylmalonsäureethylester, 6,6 g Paraformaldehyd, 30 ml Pyridin und 2 ml Piperidin 20,65 g 2-Methylen-7-phenylheptansäureethylester als Öl vom Kp. 122-125 °C bei 0,07 Torr (9,31 Pa).

c) 5-Phenylpentylmalonsäureethylester

Nach der in Beispiel 3c) beschriebenen Arbeitsweise erhält man aus 60 g 5-Phenylpentylmalonsäurediethylester und 11,16 g Kaliumhydroxid in 250 ml Ethanol 44,8 g 5-Phenylpentylmalonsäureethylester als viskoses Öl.

d) 5-Phenylpentylmalonsäurediethylester

Nach der in Beispiel 5d) beschriebenen Arbeitsweise erhält man aus 80,8 g 1-Brom-5-phenylpentan, 64,1 g Malonsäurediethylester und einer Lösung von 8,2 g Natrium in 400 ml Ethanol 66,8 g 5-Phenylpentylmalonsäurediethylester als Öl vom Kp. 142-148 °C bei 0,02 Torr (2,66 Pa).

Beispiel 11

2-[5-(4-Chlorphenyl)-pentyl]-oxiran-2-carbonsäureethylester

a) 2-[5-(4-Chlorphenyl)-pentyl]-oxiran-2-carbonsäureethylester

Nach der in Beispiel 1a) beschriebenen Arbeitsweise erhält man aus 15 g 7-(4-Chlorphenyl)-2-methylenheptansäureethylester und 23 g m-Chlorperbenzoesäure in 180 ml Methylenchlorid 8,8 g der Titelverbindung vom Kp. 135-140 °C bei 0,005 Torr (0,66 Pa).

b) 7-(4-Chlorphenyl)-2-methylenheptansäureethylester

Nach der in Beispiel 1b) beschriebenen Arbeitsweise erhält man aus 25 g 5-(4-Chlorphenyl)-pentylmalonsäureethylester, 3,8 g Paraformaldehyd, 16 ml Pyridin und 1 ml Piperidin 15,5 g 7-(4-Chlorphenyl)-2-methylenheptansäureethylester vom Kp. 140-145 °C bei 0,008 Torr (1,06 Pa).

c) 5-(4-Chlorphenyl)-pentylmalonsäureethylester

Nach der in Beispiel 3c) beschriebenen Arbeitsweise erhält man aus 34 g 5-(4-Chlorphenyl)-pentylmalonsäurediethylester und 5,7 g Kaliumhydroxid in 150 ml Ethanol 26,5 g 5-(4-Chlorphenyl)-pentylmalonsäureethylester als viskoses Öl.

d) 5-(4-Chlorphenyl)-pentylmalonsäurediethylester

Nach der in Beispiel 5d) beschriebenen Arbeitsweise erhält man aus 70,5 g p-Toluolsulfonsäure-[5-(4-chlorphenyl)-pentyl]-ester, 32 g Malonsäurediethylester und einer Lösung von 4,6 g Natrium in 250 ml Ethanol 35,5 g 5-(4-Chlorphenyl)-pentylmalonsäurediethylester als Öl.

Beispiel 12

2-[3-(4-Chlorphenyl)-propyl]-oxiran-2-carbonsäure

Nach der in Beispiel 2 beschriebenen Arbeitsweise erhält man aus 1,0 g 2-[3-(4-Chlorphenyl)-propyl]-oxiran-2-carbonsäureethylester und 3,72 ml 1n Natronlauge die Titelverbindung als zähes Öl.

Beispiel 13

2-(3-Phenylpropyl)-oxiran-2-carbonsäuremethylester

Die Titelverbindung erhält man analog Beispiel 3a) als farbloses Öl vom Kp. 75-80 °C bei 0,01 Torr (1,33 Pa).

Die Ausgangsverbindung 5-Phenyl-2-methylenvaleriansäuremethylester erhält man aus 3-Phenylpropylmalonsäuredimethylester analog Beispiel 3b) und 3c).

Beispiel 14

Natrium-2-(3-phenylpropyl)-oxiran-2-carboxylat

5 g 2-(3-Phenylpropyl)-oxiran-2-carbonsäure wird in der äquivalenten Menge 1n Natronlauge gelöst, die wässerige Lösung wird einmal mit Diethylether gewaschen und zur Trockne eingedampft. Der bei 20 °C im Vakuum getrocknete Rückstand besteht aus dem reinen Natriumsalz als glasiger Masse.

Beispiel 15

2-Benzyloxiran-2-carbonsäureethylester

Nach der in Beispiel 1a beschriebenen Arbeitsweise erhält man aus 15,0 g 2-Methylen-3-phenylpropionsäureethylester und 32,0 g m-Chlorperbenzoesäure in 300 ml Methylenchlorid 7,6 g der Titelverbindung vom Kp. 79-80 °C bei 0,003 Torr (0,4 Pa).

Beispiel 16

2-(2-Phenylethyl)-oxiran-2-carbonsäureethylester

9 g 4-Phenyl-2-methylenbuttersäureethylester und 22,3 g m-Chlorperbenzoesäure (85 %ig) werden in 150 ml Methylenchlorid 24 Stunden unter Rückfluß gekocht. Man läßt abkühlen, filtriert von der abgeschiedenen m-Chlorbenzoesäure ab, engt das Filtrat ein, nimmt den Rückstand mit 30 ml Aceton auf, gibt 15 ml einer gesättigten Natriumcarbonatlösung hinzu und rührt 1 Stunde bei 0 °C. Man verdünnt mit 100 ml Wasser, extrahiert 3 mal mit je 50 ml Methylenchlorid, engt die organische Phase ein und destilliert den öligen Rückstand. Man erhält 7,3 g der Titelverbindung vom Kp. 110-112 °C bei 0,01 Torr (1,33 Pa).

Beispiel 17

Natrium-2-[3-(3-chlorphenyl)-propyl]-oxiran-2-carboxylat

2,7 g 2-[3-(3-Chlorphenyl)-propyl]-oxiran-2-carbonsäureethylester werden mit 10 ml 1n Natronlauge und 10 ml Ethanol 1 Stunde bei Raumtemperatur gerührt. Man engt im Vakuum ein, wobei die Titelverbindung als farbloses, glasiges Pulver zurückbleibt.

Beispiel 18

2-[3-(2-Chlorphenyl)-propyl]-oxiran-2-carbonsäureethylester

Zu einer bei – 78 °C zusammengerührten Mischung aus 104 ml 15 %igem Butyllithium (in Hexan), 19 g Diisopropylamin und 400 ml Tetrahydrofuran tropft man 39 g 5-(2-Chlorphenyl)-2-acetylvaleriansäureethylester [hergestellt aus 3-(2-Chlorphenyl)-propyltosylat und Acetessigester] und rührt weitere 20 Minuten. Nach Entfernen des Kühlbades gibt man portionsweise 19,4 g Paraformaldehyd (wasserfrei)

dazu. Nach 1 Stunde Rühren erhitzt man 5 Stunden unter Rückfluß und läßt über Nacht stehen. Die festen Bestandteile werden abfiltriert, das Filtrat wird am Rotationsverdampfer eingeengt. Den Rückstand versetzt man mit 500 ml gesättigter Natriumhydrogencarbonatlösung und rührt 30 Minuten. Extraktion mit Methylenchlorid und Verdampfen des Lösungsmittels ergeben nach Reinigung durch Destillation 14 g 5-(2-Chlorphenyl)-2-methylenvaleriansäureethylester vom Kp. 95-98 °C bei 0,005 Torr (0,66 Pa). Die Oxidation zur Titelverbindung erfolgt analog Beispiel 4a).

Beispiel 19

Natrium-2-[3-(4-chlorphenyl)-propyl]-oxiran-2-carboxylat

Zu einer Lösung von 0,36 g Natrium in 15 ml Ethanol tropft man eine Lösung von 4,2 g 2-[3-(4-Chlorphenyl)-propyl]-oxiran-2-carbonsäureethylester, gibt anschließend 0,28 g Wasser zu und rührt 1 Stunde bei Raumtemperatur. Der ausgefallene Niederschlag wird abfiltriert, mit Diethylether gewaschen und einmal aus Ethanol/Diethylether umkristallisiert. Man erhält 2,0 g der Titelverbindung vom F. 160-167 °C.

Beispiel 20

Natrium-2-[3-(3-trifluormethylphenyl)-propyl]-oxiran-2-carboxylat

Zu einer Lösung aus 92,6 ml 1n Natronlauge und 50 ml Tetrahydrofuran tropft man bei Raumtemperatur eine Lösung von 28 g 2-[3-(3-Trifluormethylphenyl)-propyl]-oxiran-2-carbonsäureethylester in 50 ml Tetrahydrofuran. Nach der Bildung einer klaren Lösung wird die Lösung im Vakuum zu einer zähen Masse eingeengt. Man löst in 70 ml Tetrahydrofuran, filtriert von wenig ungelöstem Material und versetzt das klare Filtrat tropfenweise mit 200 ml Dichlormethan. Der voluminöse Niederschlag wird abfiltriert, nochmals mit 200 ml Diethylether verrührt, abgesaugt und über Calciumchlorid getrocknet. Die 2 Mol Kristallwasser enthaltende Titelverbindung vom F. 64-70 °C geht beim Trocknen über Phosphorpentoxid im Vakuum in die kristallwasserfreie Form vom F. > 170 °C über (Ausbeute 22,8 g).

Beispiel 21

Natrium-2-(5-phenylpentyl)-oxiran-2-carboxylat

Zu einer Lösung von 43,6 g 2-(5-Phenylpentyl)-oxiran-2-carbonsäureethylester in 90 ml Tetrahydrofuran tropft man eine Mischung aus 166 ml 1n Natronlauge und 90 ml Tetrahydrofuran. Nach beendeter Zugabe rührt man ca. 45 Minuten, bis eine klare Lösung entsteht. Innerhalb 3 Stunden werden nunmehr 1,6 l Aceton zugetropft, wobei ein kristalliner Niederschlag ausfällt, der abfiltriert und mit Aceton gewaschen wird. Man kristallisiert aus Wasser um und erhält 42,9 g der Titelverbindung als Dihydrat vom F. 82-86 °C.

Beispiel 22

Natrium-2-[5-(4-chlorphenyl)-pentyl]-oxiran-2-carboxylat

Eine Mischung aus 8,05 g 2-[5-(4-Chlorphenyl)-pentyl]-oxiran-2-carbonsäureethylester, 27 ml Tetrahydrofuran und 27 ml 1n Natronlauge wird ca. 1 Stunde bis zur Bildung einer klaren Lösung gerührt. Man engt ein und kristallisiert den farblosen Rückstand aus Ethanol/Diethylether um. Man erhält 6,5 g der Titelverbindung vom F. 136-142 °C.

Beispiel 23

Natrium-2-[3-(4-methylphenyl)-propyl]-oxiran-2-carboxylat

Nach der in Beispiel 19 beschriebenen Arbeitsweise erhält man aus einer Lösung von 0,7 g Natrium in 70 ml Ethanol, 0,5 ml Wasser und 7,0 g 2-[3-(4-Methylphenyl)-propyl]-oxiran-2-carbonsäureethylester 2,7 g der Titelverbindung vom F. > 250 °C (aus Methanol/Diethylether).

Beispiel 24

2-[3-(4-Fluorphenyl)-propyl]-oxiran-2-carbonsäureethylester

a) 2-[3-(4-Fluorphenyl)-propyl]-oxiran-2-carbonsäureethylester

Nach der in Beispiel 1a beschriebenen Arbeitsweise erhält man aus 25,4 g 5-(4-Fluorphenyl)-2-methylenvaleriansäureethylester und 32,2 g m-Chlorperbenzoesäure in 330 ml Methylenchlorid 23,8 g

der Titelverbindung vom Kp. 115-117 °C bei 0,02 Torr (2,66 Pa).

b) 5-(4-Fluorphenyl)-2-methylenvaleriansäureethylester

Nach der in Beispiel 1b beschriebenen Arbeitsweise erhält man aus 43,5 g 3-(4-Fluorphenyl)-propylmalonsäureethylester, 5,1 g Paraformaldehyd, 50 ml Pyridin und 1,6 ml Piperidin · 25,7 g 5-(4-Fluorphenyl)-2-methylenvaleriansäureethylester vom Kp. 84-86 °C bei 0,01 Torr (1,33 Pa).

c) 3-(4-Fluorphenyl)-propylmalonsäureethylester

Nach der in Beispiel 3c beschriebenen Arbeitsweise erhält man aus 80 g 3-(4-Fluorphenyl)-propylmalonsäurediethylester und 16,6 g Kaliumhydroxid in 170 ml Ethanol 43,5 g 3-(4-Fluorphenyl)-propylmalonsäureethylester als gelbliches Öl.

d) 3-(4-Fluorphenyl)-propylmalonsäurediethylester

Nach der in Beispiel 5d beschriebenen Arbeitsweise erhält man aus 83 g p-Toluolsulfonsäure-[3-(4-fluorphenyl)-propyl]-ester, 43,3 g Malonsäurediethylester und einer Lösung von 6,5 g Natrium in 400 ml Ethanol 80 g 3-(4-Fluorphenyl)-propylmalonsäurediethylester als gelbliches Öl.

e) p-Toluolsulfonsäure-[3-(4-fluorphenyl)-propyl]-ester

Nach der in Beispiel 5e beschriebenen Arbeitsweise erhält man aus 39,7 g 3-(4-Fluorphenyl)-propanol-1,54 g p-Toluolsulfonsäurechlorid und 83 ml Pyridin in 200 ml Toluol nach 40 Stunden Rühren bei Raumtemperatur 83 g p-Toluolsulfonsäure-[3-(4-fluorphenyl)-propyl]-ester als gelbliches Öl.

f) 3-(4-Fluorphenyl)-propanol-1

Zu einer Suspension von 19,7 g Lithiumaluminiumhydrid in 300 ml Tetrahydrofuran tropft man bei ca. 45 °C Reaktionstemperatur unter Rühren eine Lösung von 43,6 g 3-(4-Fluorphenyl)-propionsäure in 300 ml Tetrahydrofuran. Nach beendeter Zugabe hält man noch 2,5 Stunden bei dieser Temperatur, tropft dann nacheinander 80 ml Wasser und 20 ml 4n Natronlauge zu. Man filtriert den Niederschlag ab, wäscht mehrmals mit Diethylether nach, trocknet die vereinigten Lösungen über Natriumsulfat und engt ein. Zurück bleiben 39,7 g 3-(4-Fluorphenyl)-propanol-1 als fast farbloses Öl.

g) 3-(4-Fluorphenyl)-propionsäure

Zu einer Lösung von 12,6 g Natrium in 300 ml Ethanol tropft man 91,6 g Malonsäurediethylester, rührt noch 15 Minuten nach und tropft dann 98,3 g 4-Fluorbenzylbromid zu. Man kocht anschließend noch 3 Stunden unter Rückfluß, destilliert den größten Teil des Lösungsmittels ab, nimmt den Rückstand mit Eiswasser (800 ml) und Methylenchlorid (600 ml) auf und schüttelt gut durch. Die organische Phase wird gesammelt, eingeengt und das zurückbleibende Öl (4-Fluorbenzylmalonsäurediethylester) (137,6 g) mit einer Lösung von 133 g Kaliumhydroxid in 780 ml Methanol 12 Stunden gerührt. Man engt im Vakuum weitgehend ein, löst den Rückstand in Wasser/Diethylether, schüttelt gut durch, trennt die organische Phase ab und säuert die wäßrige Phase unter Eiskühlung mit 10n Schwefelsäure an. Man extrahiert mit Methylenchlorid, engt die organische Phase ein und verrührt den öligen Rückstand mit Petrolether/Essigester (3 : 1), wobei 53,6 g 4-Fluorbenzylmalonsäure auskristallisieren (F. 134-136 °C).

Die 4-Fluorbenzylmalonsäure wird 1,5 Stunden lang auf 170-175 °C erhitzt. Das Reaktionsprodukt wird nach dem Abkühlen mit wenig Diethylether verrührt. Hierbei kristallisieren 41,6 g 3-(4-Fluorphenyl)-propionsäure vom F. 85-88 °C [aus Essigester/Petrolether (1 : 4)] aus.

## Beispiel 25

2-(6-Phenylhexyl)-oxiran-2-carbonsäureethylester

a) 2-(6-Phenylhexyl)-oxiran-2-carbonsäureethylester

Nach der in Beispiel 1a beschriebenen Arbeitsweise erhält man aus 4 g 2-Methylen-8-phenyloctansäureethylester und 5,4 g m-Chlorperbenzoesäure in 150 ml Methylenchlorid 2,1 g der Titelverbindung als farbloses Öl, das durch Chromatographie an einer Kieselgelsäule [Laufmittel : Petrolether/Essigsäureethylester (95 : 5)] gereinigt wird.

b) 2-Methylen-8-phenyloctansäureethylester

Nach der in Beispiel 1b beschriebenen Arbeitsweise erhält man aus 7 g 6-Phenylhexylmalonsäureethylester, 0,8 g Paraformaldehyd, 10 ml Pyridin und 0,5 ml Piperidin 4,0 g 2-Methylen-8-phenyl-

octansäureethylester als farbloses Öl, das über eine Kieselgelsäule (Laufmittel: Methylenchlorid) gereinigt wird.

c) 6-Phenylhexylmalonsäureethylester

Nach der in Beispiel 3c beschriebenen Arbeitsweise erhält man aus 20 g 6-Phenylhexylmalonsäurediethylester und Kaliumhydroxid 14,5 g 6-Phenylhexylmalonsäureethylester als zähes Öl.

d) 6-Phenylhexylmalonsäurediethylester

Nach der in Beispiel 5d beschriebenen Arbeitsweise erhält man aus 24 g 6-Phenylhexylbromid, 24 g Malonsäurediethylester und einer Lösung von 2,6 g Natrium in 150 ml Ethanol 22 g 6-Phenylhexylmalonsäurediethylester als helles Öl.

Beispiel 26

2-(5-Phenylpentyl)-oxiran-2-carbonsäure

3 g Natrium-2-(5-phenylpentyl)-oxiran-2-carboxylat werden mit 70 ml eiskalter 1n Salzsäure und 50 ml Diethylether durchgeschüttelt; die organische Phase wird gesammelt, über Natriumsulfat getrocknet und eingeengt. Zurück bleiben 1,7 g der Titelverbindung als zähes, farbloses Öl.

Beispiel 27

2-(7-Phenylheptyl)-oxiran-2-carbonsäureethylester

a) 2-(7-Phenylheptyl)-oxiran-2-carbonsäureethylester

Nach der in Beispiel 1a beschriebenen Arbeitsweise erhält man aus 4,88 g 2-Methylen-9-phenylnonansäureethylester und 6,2 g m-Chlorperbenzoesäure 1,7 g der Titelverbindung vom Kp. 126-132 °C bei 0,15 Torr (2 Pa).

b) 2-Methylen-9-phenylnonansäureethylester

Nach der in Beispiel 1b beschriebenen Arbeitsweise erhält man aus 7,77 g 7-Phenylheptylmalonsäureethylester, 1,08 g Paraformaldehyd, 4,8 ml Pyridin und 0,32 ml Piperidin 5,88 g 2-Methylen-9-phenylnonansäureethylester vom Kp. 136 °C bei 0,05 Torr (6,65 Pa).

c) 7-Phenylheptylmalonsäureethylester

Nach der in Beispiel 3c beschriebenen Arbeitsweise erhält man aus 10,2 g 7-Phenylheptylmalonsäurediethylester und 1,71 g Kaliumhydroxid in 30 ml Ethanol 7,77 g 7-Phenylheptylmalonsäureethylester als zähes Öl.

d) 7-Phenylheptylmalonsäurediethylester

Nach der in Beispiel 5d beschriebenen Arbeitsweise erhält man aus 17 g 7-Phenylheptylbromid, 16 g Malonsäurediethylester und einer Lösung von 2,0 g Natrium in 100 ml Ethanol 12,1 g 7-Phenylheptylmalonsäurediethylester als farbloses Öl.

Beispiel 28

2-[3-(3,4-Dichlorphenyl)-propyl]-oxiran-2-carbonsäureethylester

a) 2-[3-(3,4-Dichlorphenyl)-propyl]-oxiran-2-carbonsäureethylester

Nach der in Beispiel 1a beschriebenen Arbeitsweise erhält man aus 33,1 g 5-(3,4-Dichlorphenyl)-2-methylenvaleriansäureethylester und 45,6 g m-Chlorperbenzoesäure 27,0 g der Titelverbindung als farbloses Öl, das durch Chromatographie über eine Kieselgelsäule (Laufmittel: Methylenchlorid) gereinigt wird.

b) 5-(3,4-Dichlorphenyl)-2-methylenvaleriansäureethylester

Nach der in Beispiel 1b beschriebenen Arbeitsweise erhält man aus 53,9 g 3-(3,4-Dichlorphenyl)-propylmalonsäureethylester, 5,4 g Paraformaldehyd, 55 ml Pyridin und 1,7 ml Piperidin 33,1 g 5-(3,4-Dichlorphenyl)-2-methylenvaleriansäureethylester vom Kp. 128-130 °C bei 0,02 Torr (2,66 Pa).

c) 3-(3,4-Dichlorphenyl)-propylmalonsäureethylester

Nach der in Beispiel 3c beschriebenen Arbeitsweise erhält man aus 81 g 3-(3,4-Dichlorphenyl)-propylmalonsäurediethylester und 14,8 g Kaliumhydroxid in 340 ml Ethanol 53,9 g 3-(3,4-Dichlorphenyl)-propylmalonsäureethylester als helles Öl.

d) 3-(3,4-Dichlorphenyl)-propylmalonsäurediethylester

Nach der in Beispiel 5d beschriebenen Arbeitsweise erhält man aus 82,1 g p-Toluolsulfonsäure-[3-(3,4-dichlorphenyl)-propyl]-ester, 38,4 g Malonsäurediethylester und einer Lösung von 5,3 g Natrium in 400 ml Ethanol 79 g 3-(3,4-Dichlorphenyl)-propylmalonsäurediethylester als gelbliches Öl.

e) p-Toluolsulfonsäure-[3-(3,4-dichlorphenyl)-propyl]-ester

Nach der in Beispiel 5e beschriebenen Arbeitsweise erhält man aus 125 g 3-(3,4-Dichlorphenyl)-propanol-1, 117,4 g p-Toluolsulfonsäurechlorid und 200 ml Pyridin in 600 ml Toluol 175,4 g p-Toluolsulfonsäure-[3-(3,4-dichlorphenyl)-propyl]-ester als zähes Öl.

Beispiel 29

2-[3-(3,4-Dichlorphenyl)-propyl]-oxiran-2-carbonsäuremethylester

8,0 g der Titelverbindung erhält man analog Beispiel 1a aus 19,2 g 5-(3,4-Dichlorphenyl)-2-methylenvaleriansäuremethylester und 27,5 g m-Chlorperbenzoesäure als farbloses Öl, das durch Chromatographie an Kieselgel [Laufmittel : Methylenchlorid/Petrolether (1 : 1)] gereinigt wird.

Die Ausgangsverbindung 5-(3,4-Dichlorphenyl)-2-methylenvaleriansäuremethylester (Öl) erhält man aus 3-(3,4-Dichlorphenyl)-propylmalonsäuredimethylester analog Beispiel 3b und 3c.

Beispiel 30

2-[3-(5-Chlor-2-methoxyphenyl)-propyl]-oxiran-2-carbonsäureethylester

a) 2-[3-(5-Chlor-2-methoxyphenyl)-propyl]-oxiran-2-carbonsäureethylester

Nach der in Beispiel 1a beschriebenen Arbeitsweise erhält man aus 19,5 g 5-(5-Chlor-2-methoxyphenyl)-2-methylenvaleriansäureethylester und 28 g m-Chlorperbenzoesäure in 250 ml Methylenchlorid 8,8 g der Titelverbindung als farbloses Öl vom Kp. 150 °C bei 0,005 Torr (0,66 Pa).

b) 5-(5-Chlor-2-methoxyphenyl)-2-methylenvaleriansäureethylester

Nach der in Beispiel 1b beschriebenen Arbeitsweise erhält man aus 28,5 g 3-(5-Chlor-2-methoxyphenyl)-propylmalonsäureethylester, 3,2 g Paraformaldehyd, 16,3 ml Pyridin und 1,1 ml Piperidin 20,3 g 5-(5-Chlor-2-methoxyphenyl)-2-methylenvaleriansäureethylester vom Kp. 136-140 °C bei 0,01 Torr (1,33 Pa).

c) 3-(5-Chlor-2-methoxyphenyl)-propylmalonsäureethylester

Nach der in Beispiel 3c beschriebenen Arbeitsweise erhält man aus 34,8 g 3-(5-Chlor-2-methoxyphenyl)-propylmalonsäurediethylester und 6,4 g Kaliumhydroxid in 400 ml Ethanol 28,5 g 3-(5-Chlor-2-methoxyphenyl)-propylmalonsäureethylester als zähes Öl.

d) 3-(5-Chlor-2-methoxyphenyl)-propylmalonsäurediethylester

Nach der in Beispiel 5d beschriebenen Arbeitsweise erhält man aus 38 g 3-(5-Chlor-2-methoxyphenyl)-propylchlorid, 44 g Malonsäurediethylester und einer Lösung von 3,9 g Natrium in 150 ml Ethanol 19,1 g 3-(5-Chlor-2-methoxyphenyl)-propylmalonsäurediethylester vom Kp. 136-145 °C bei 0,01 Torr (1,33 Pa).

e) 3-(5-Chlor-2-methoxyphenyl)-propylchlorid

Es werden 58,2 g 3-(5-Chlor-2-methoxyphenyl)-propanol-1 und 50 ml Thionylchlorid 8 Stunden bei 50 °C gerührt ; das überschüssige Thionylchlorid im Vakuum abdestilliert und der Rückstand im Hochvakuum destilliert. Man erhält 50,9 g 3-(5-Chlor-2-methoxyphenyl)-propylchlorid vom Kp. 87-95 °C bei 0,005 Torr (0,66 Pa).

f) 3-(5-Chlor-2-methoxyphenyl)-propanol-1

Nach der in Beispiel 24f beschriebenen Arbeitsweise erhält man aus 96,6 g 3-(5-Chlor-2-methoxyphenyl)-propionsäure und 14 g Lithiumaluminiumhydrid in 900 ml Diethylether 66,7 g 3-(5-Chlor-2-methoxyphenyl)-propanol-1 vom Kp. 94-97 °C bei 0,001 Torr (0,13 Pa).

g) 3-(5-Chlor-2-methoxyphenyl)-propionsäure

Nach der in Beispiel 24g beschriebenen Arbeitsweise erhält man aus 100 g 5-Chlor-2-methoxybenzylchlorid, 120 ml Malonsäurediethylester und einer Lösung von 12,07 g Natrium in 1,1 l Ethanol 124 g 5-Chlor-2-methoxybenzylmalonsäurediethylester, der nach Verseifung mit Kaliumhydroxid und Erhitzen der entstandenen 5-Chlor-2-methoxybenzylmalonsäure auf 160-170 °C 63,2 g 3-(5-Chlor-2-methoxyphenyl)-propionsäure vom F. 91-92 °C ergibt.

Beispiel 31

2-(8-Phenyloctyl)-oxiran-2-carbonsäureethylester

a) 2-(8-Phenyloctyl)-oxiran-2-carbonsäureethylester

Nach der in Beispiel 1a beschriebenen Arbeitsweise erhält man aus 11,3 g 2-Methylen-10-phenyldecansäureethylester und 16 g m-Chlorperbenzoesäure in 300 ml Methylenchlorid 10,4 g der Titelverbindung als farbloses Öl, das durch Chromatographie an Kieselgel (Laufmittel : Methylenchlorid) gereinigt wird.

b) 2-Methylen-10-phenyldecansäureethylester

Nach der in Beispiel 1b beschriebenen Arbeitsweise erhält man aus 16,5 g 8-Phenyloctylmalonsäureethylester, 1,65 g Paraformaldehyd, 20 ml Pyridin und 0,5 ml Piperidin 11,4 g 2-Methylen-10-phenyldecansäureethylester als farbloses Öl, das durch Chromatographie an Kieselgel (Laufmittel : Chloroform) gereinigt wird.

c) 8-Phenyloctylmalonsäureethylester

Nach der in Beispiel 3c beschriebenen Arbeitsweise erhält man aus 25,3 g 8-Phenyloctylmalonsäurediethylester und 4,6 g Kaliumhydroxid in 150 ml Ethanol 16,7 g 8-Phenyloctylmalonsäureethylester als zähes Öl.

d) 8-Phenyloctylmalonsäurediethylester

Nach der in Beispiel 5d beschriebenen Arbeitsweise erhält man aus 17,4 g 8-Phenyloctylchlorid, 13 g Malonsäurediethylester, einer Lösung von 1,8 g Natrium in 70 ml Ethanol und einer Spatelspitze Kaliumiodid 25,3 g 8-Phenyloctylmalonsäurediethylester als gelbliches Öl.

e) 8-Phenyloctylchlorid

Nach der in Beispiel 30e beschriebenen Arbeitsweise erhält man aus 16,35 g 8-Phenyloctanol-1 und 16 ml Thionylchlorid 17,4 g 8-Phenyloctylchlorid als helles Öl.

Beispiel 32

2-[3-(4-tert.-Butylphenyl)-propyl]-oxiran-2-carbonsäuremethylester

a) 2-[3-(4-tert.-Butylphenyl)-propyl]-oxiran-2-carbonsäuremethylester

Nach der in Beispiel 1a beschriebenen Arbeitsweise erhält man aus 13 g 5-(4-tert.-Butylphenyl)-2-methylenvaleriansäuremethylester und 17,7 g m-Chlorperbenzoesäure in 300 ml Methylenchlorid 10,7 g der Titelverbindung als farbloses Öl, das durch Chromatographie an Kieselgel [Laufmittel : Petrolether/Essigsäureethylester (90 : 10)] gereinigt wird.

b) 5-(4-tert.-Butylphenyl)-2-methylenvaleriansäuremethylester

Nach der in Beispiel 1b beschriebenen Arbeitsweise erhält man aus 18 g 3-(4-tert.-Butylphenyl)-propylmalonsäuremethylester, 1,94 g Paraformaldehyd, 20 ml Pyridin und 0,5 ml Piperidin 13,1 g 5-(4-tert.-Butylphenyl)-2-methylenvaleriansäuremethylester als farbloses Öl, das durch Chromatographie an

Kieselgel (Laufmittel : Methylenchlorid) gereinigt wird.

c) 3-(4-tert.-Butylphenyl)-propylmalonsäuremethylester

Nach der in Beispiel 3c beschriebenen Arbeitsweise erhält man aus 20,9 g 3-(4-tert.-Butylphenyl)-propylmalonsäuredimethylester und 4,5 g Kaliumhydroxid in 140 ml Methanol 18,0 g 3-(4-tert.-Butylphenyl)-propylmalonsäuremethylester als zähes Öl.

d) 3-(4-tert.-Butylphenyl)-propylmalonsäuredimethylester

Nach der in Beispiel 5d beschriebenen Arbeitsweise erhält man aus 64,9 g p-Toluolsulfonsäure-[3-(4-tert.-butylphenyl)-propyl]-ester, 26 g Malonsäuredimethylester und einer Lösung von 4,74 g Natrium in 250 ml Methanol 51,8 g 3-(4-tert.-Butylphenyl)-propylmalonsäuredimethylester als gelbliches Öl.

e) p-Toluolsulfonsäure-[3-(4-tert.-butylphenyl)-propyl]-ester

Nach der in Beispiel 5e beschriebenen Arbeitsweise erhält man aus 33 g 3-(4-tert.-Butylphenyl)-propanol-1, 38,5 g p-Toluolsulfonsäurechlorid und 65 ml Pyridin in 200 ml Toluol 64,9 g p-Toluolsulfonsäure-[3-(4-tert.-butylphenyl)-propyl]-ester als hellgelbes Öl.

## Beispiel 33

2-[7-(4-Chlorphenyl)-heptyl]-oxiran-2-carbonsäureethylester

a) 2-[7-(4-Chlorphenyl)-heptyl]-oxiran-2-carbonsäureethylester

Nach der in Beispiel 1a beschriebenen Arbeitsweise erhält man aus 10,0 g 9-(4-Chlorphenyl)-2-methylennonansäureethylester und 13,15 g m-Chlorperbenzoesäure in 50 ml Methylenchlorid 6,13 g der Titelverbindung vom Kp. 145-148 °C bei 0,005 Torr (0,66 Pa).

b) 9-(4-Chlorphenyl)-2-methylennonansäureethylester

Nach der in Beispiel 1b beschriebenen Arbeitsweise erhält man aus 33 g 7-(4-Chlorphenyl)-heptylmalonsäureethylester, 4,1 g Paraformaldehyd, 17,8 ml Pyridin und 1,2 ml Piperidin 15,9 g 9-(4-Chlorphenyl)-2-methylennonansäureethylester als farbloses Öl vom Kp. 134-136 °C bei 0,005 Torr (0,66 Pa).

c) 7-(4-Chlorphenyl)-heptylmalonsäureethylester

Nach der in Beispiel 3c beschriebenen Arbeitsweise erhält man aus 37,37 g 7-(4-Chlorphenyl)-heptylmalonsäurediethylester und 6,56 g Kaliumhydroxid in 100 ml Ethanol 33,62 g 7-(4-Chlorphenyl)-heptylmalonsäureethylester als viskoses Öl.

d) 7-(4-Chlorphenyl)-heptylmalonsäurediethylester

Nach der in Beispiel 5d beschriebenen Arbeitsweise erhält man aus 33,0 g 7-(4-Chlorphenyl)-heptylbromid, 27,36 g Malonsäurediethylester und einer Lösung von 2,62 g Natrium in 200 ml Ethanol 37,37 g 7-(4-Chlorphenyl)-heptylmalonsäurediethylester vom Kp. 160-165 °C bei 0,005 Torr (0,66 Pa).

e) 7-(4-Chlorphenyl)-heptylbromid

Es werden 30 g 7-(4-Chlorphenyl)-heptanol-1, 0,13 g roten Phosphor und 37 ml 62 % Bromwasserstoff 6 Stunden gekocht, dann 8 ml konzentrierte Schwefelsäure zugetropft und nochmals 6 Stunden gekocht. Die Reaktionsmischung wird in 100 ml Eiswasser gegossen und 2 mal mit Diethylether extrahiert ; die Etherextrakte werden eingeengt, und der Rückstand wird destilliert. Man erhält 33,5 g 7-(4-Chlorphenyl)-heptylbromid vom Kp. 125-127 °C bei 0,1 Torr (13,3 Pa).

f) 7-(4-Chlorphenyl)-heptanol

Nach der in Beispiel 24f beschriebenen Arbeitsweise erhält man aus 51 g 7-(4-Chlorphenyl)-heptansäure und 8,0 g Lithiumaluminiumhydrid in 500 ml Diethylether 31,2 g 7-(4-Chlorphenyl)-heptanol vom Kp. 140 °C bei 0,3 Torr (40 Pa).

g) 7-(4-Chlorphenyl)-heptansäure

80 g 5-(4-Chlorphenyl)-pentylmalonsäureethylester werden mit 45 g Kaliumhydroxid in 230 ml

Wasser und 110 ml Ethanol 5 Stunden unter Rückfluß gekocht; nach dem Erkalten wird mit konzentrierter Salzsäure auf pH 1-2 eingestellt und mit Diethylether 3 mal extrahiert. Die vereinigten organischen Lösungen werden eingeengt und der Rückstand [5-(4-Chlorphenyl)-pentylmalonsäure] 3,5 Stunden auf 160 °C erhitzt. Der Rückstand besteht aus 51 g 7-(4-Chlorphenyl)-heptansäure vom F. 75-78 °C.

Beispiel 34

Calcium-2-(5-phenylpentyl)-oxiran-2-carboxylat

Zu einer Lösung von 1,0 g Natrium-2-(5-phenylpentyl)-oxiran-2-carboxylat in 30 ml Wasser gibt man eine Lösung von 400 mg Calciumchlorid in 5 ml Wasser. Man reibt gut durch, dekantiert die Lösung von dem ausgefallenen zähen Niederschlag, reibt mit Wasser durch und dekantiert erneut. Man erhält nach dem Trocknen über Phosphorpentoxid 820 mg der Titelverbindung, die bei 270 °C erweicht und bei ca. 300 °C unter Zersetzung schmilzt.

Beispiel 35

2-(3-Phenylpropyl)-oxiran-2-carbonsäure-sec.-butylester

a) 2-(3-Phenylpropyl)-oxiran-2-carbonsäure-sec.-butylester

Nach der in Beispiel 1a) beschriebenen Arbeitsweise erhält man aus 10 g 2-Methylen-5-phenylvaleriansäure-sec.-butylester und 18 g m-Chlorperbenzoesäure in 200 ml Methylenchlorid 6,1 g der Titelverbindung als fast farbloses Öl [gereinigt durch Chromatographie an Kieselgel (Laufmittel: Petrolether/Essigsäureethylester 9 : 1)].

b) 2-Methylen-5-phenylvaleriansäure-sec.-butylester

27 g 2-Methylen-5-phenylvaleriansäurechlorid werden bei 25-30 °C zu einer Lösung von 100 ml sec.-Butylalkohol und 20 ml Triethylamin in 300 ml Diethylether getropft. Nach beendeter Zugabe rührt man noch 1 Stunde bei Raumtemperatur nach, gibt 500 ml Eiswasser zu, schüttelt durch und sammelt die organische Phase. Nach dem Trocknen der organischen Phase über Natriumsulfat und Einengen erhält man 19,5 g 2-Methylen-5-phenylvaleriansäure-sec.-butylester vom Kp. 115-126 °C bei 0,01 Torr (1,3 Pa).

c) 2-Methylen-5-phenylvaleriansäurechlorid

25 g 2-Methylen-5-phenylvaleriansäure und 20 ml frisch destilliertes Thionylchlorid werden 6 Stunden bei 50 °C gerührt. Anschließend wird das überschüssige Thionylchlorid im Vakuum abdestilliert. Zurück bleiben 27 g 2-Methylen-5-phenylvaleriansäurechlorid als braune Flüssigkeit, die ohne Reinigung weiterverarbeitet werden.

Beispiel 36

2-[3-(4-Chlorphenyl)-propyl]-oxiran-2-carbonsäureisopropylester

a) 2-[3-(4-Chlorphenyl)-propyl]-oxiran-2-carbonsäureisopropylester

Nach der in Beispiel 1a) beschriebenen Arbeitsweise erhält man aus 12 g 5-(4-Chlorphenyl)-2-methylenvaleriansäureisopropylester und 20 g m-Chlorperbenzoesäure in 150 ml Methylenchlorid 5,4 g der Titelverbindung als gelbliches Öl [gereinigt durch Chromatographie an Kieselgel (Laufmittel: Petrolether/Essigsäureethylester 9 : 1)].

b) 5-(4-Chlorphenyl)-2-methylenvaleriansäureisopropylester

Nach der in Beispiel 35b) beschriebenen Arbeitsweise erhält man aus 21,5 g 5-(4-Chlorphenyl)-2-methylenvaleriansäurechlorid, 100 ml Isopropanol und 18 ml Triethylamin in 300 ml Diethylether 12,2 g 5-(4-Chlorphenyl)-2-methylenvaleriansäureisopropylester vom Kp. 128-134 °C bei 0,01 Torr (1,3 Pa).

c) 5-(4-Chlorphenyl)-2-methylenvaleriansäurechlorid

Nach der in Beispiel 35c) beschriebenen Arbeitsweise erhält man aus 20,5 g 5-(4-Chlorphenyl)-2-methylenvaleriansäure und 20 ml Thionylchlorid 21,5 g 5-(4-Chlorphenyl)-2-methylenvaleriansäurechlorid als braunes Öl.

d) 5-(4-Chlorphenyl)-2-methylenvaleriansäure

25 g 5-(4-Chlorphenyl)-2-methylenvaleriansäureethylester, 100 ml 2n Natronlauge und 50 ml Ethanol werden 3 Stunden bei 50 °C gerührt. Nach dem Abkühlen wird die Reaktionsmischung unter Eiskühlung langsam mit 110 ml 2n Salzsäure versetzt und 4 mal mit je 50 ml Diethylether extrahiert. Die vereinigten organischen Phasen werden mit Wasser gewaschen und nach dem Trocknen über Natriumsulfat eingeengt. Zurück bleiben 20,5 g 5-(4-Chlorphenyl)-2-methylenvaleriansäure als braunes Öl.

## Beispiel 37

Ansatz für Ampullen

1 000 g 2-[3-(4-Chlorphenyl)-propyl]-oxiran-2-carbonsäure werden in ca. 80 Liter aqua bidest. unter Zusatz der äquivalenten Menge Natronlauge gelöst. Die Lösung wird auf pH 7,0 ± 0,5 eingestellt und mit aqua bidest. auf 100 Liter aufgefüllt. Dann wird über ein Filter steril filtriert und unter keimfreien Bedingungen in 2 ml-Ampullen abgefüllt.

## Beispiel 38

100 000 Kapseln mit einem Wirkstoffgehalt von 30 mg werden aus folgenden Bestandteilen hergestellt:

3 000 g 2-(4-Phenylbutyl)-oxiran-2-carbonsäureethylester werden mit 5 000 g Neutralöl gemischt und in Weichgelatinekapseln abgefüllt.

## Beispiel 39

10 000 Kapseln mit einem Wirkstoffgehalt von 25 mg werden wie folgt hergestellt:

250 g 2-[3-(3-Chlorphenyl)-propyl)-oxiran-2-carbonsäureethylester werden in 1 000 ml Methylenchlorid gelöst. Die Lösung wird mit 750 g mikronisierter Kieselsäure gut gemischt. Die Mischung wird zur Trockne eingedampft und dann in Hartgelatinekapseln abgefüllt.

## Beispiel 40

10 000 Kapseln mit einem Wirkstoffgehalt von 20 mg werden wie folgt hergestellt:

200 g 2-[3-(3-Trifluormethylphenyl)-propyl]-oxiran-2-carbonsäureethylester werden in 1 000 ml Methylenchlorid gelöst. Die Lösung wird mit 800 g mikronisierter Kieselsäure gut gemischt. Die Mischung wird zur Trockne eingedampft und dann in Hartgelatinekapseln abgefüllt.

## Beispiel 41

10 000 Kapseln mit einem Wirkstoffgehalt von 25 mg werden wie folgt hergestellt:

250 g 2-[5-(4-Chlorphenyl)-pentyl]-oxiran-2-carbonsäureethylester werden in 1 000 ml Methylenchlorid gelöst. Die Lösung wird mit 750 g mikronisierter Kieselsäure gut gemischt. Die Mischung wird zur Trockne eingedampft und dann in Hartgelatinekapseln abgefüllt.

## Beispiel 42

Tabletten mit einem Wirkstoffgehalt von 25 mg werden wie folgt hergestellt:

10 kg Natrium-2-[5-(4-chlorphenyl)-pentyl]-oxiran-2-carboxylat, 45 kg Xylit und 30 kg Calciumphosphat werden mit 2,5 kg Polyvinylpyrrolidon (MG~25 000; MG = Molekulargewicht) in ungefähr 6 l Wasser granuliert. Das Granulat wird durch ein Sieb von 1,25 mm Maschenweite gesiebt und nach dem Trocknen werden 9 kg Carboxymethylcellulose, 2,5 kg Talkum und 1 kg Magnesiumstearat zugegeben. Man verpreßt das trockene Granulat zu Tabletten von 8 mm Durchmesser, 250 mg Gewicht und einer Härte von 5-6 kg.

## Pharmakologie

Die erfindungsgemäßen substituierten Oxirancarbonsäuren der allgemeinen Formel I senken den Blutglucosespiegel und den Blutspiegel der Ketonkörper, wobei sie sich in ihrer chemischen Struktur und ihrer Wirkungsweise grundlegend von pankreaswirksamen, betacytotropen Substanzen (z. B. Sulfonylharnstoffen) durch ihre extrapankreatische Wirkung unterscheiden und extrapankreatisch wirkenden Handelspräparaten (z. B. Buformin und Phenformin) überlegen erweisen.

In den anschließenden Tabellen werden die untersuchten Verbindungen durch eine laufende Nummer gekennzeichnet, die wie folgt zuzuordnen ist:

| Lfd. Nr. | Name der Verbindung |
|---|---|
| 1 | Buformin |
| 2 | Phenformin |
| 3 | 2-[3-(4-Chlorphenyl)-propyl]-oxiran-2-carbonsäureethylester |
| 4 | 2-[3-(4-Chlorphenyl)-propyl]-oxiran-2-carbonsäure (Na-Salz) |
| 5 | 2-[3-(3-Trifluormethylphenyl)-propyl]-oxiran-2-carbonsäureethylester |
| 6 | 2-[3-(3-Trifluormethylphenyl)-propyl]-oxiran-2-carbonsäure (Na-Salz) |
| 7 | 2-[5-Phenyl-pentyl]-oxiran-2-carbonsäureethylester |
| 8 | 2-[5-Phenyl-pentyl]-oxiran-2-carbonsäure (Na-Salz) |
| 9 | 2-[5-(4-Chlorphenyl)-pentyl]-oxiran-2-carbonsäureethylester |
| 10 | 2-[5-(4-Chlorphenyl]-pentyl]-oxiran-2-carbonsäure (Na-Salz) |
| 11 | 2-[4-Phenylbutyl]-oxiran-2-carbonsäureethylester |
| 12 | 2-[3-(3-Chlorphenyl)-propyl]-oxiran-2-carbonsäureethylester |

In Tabelle I werden Untersuchungen des Einflusses von Vertretern der erfindungsgemäßen Verbindungen auf die Blutglucosekonzentration nüchterner stoffwechselgesunder Ratten nach einmaliger oraler Substanzgabe von 0,056-0,6 mmol/kg Körpergewicht innerhalb von 5 Stunden wiedergegeben. In Spalte A ist die Wirkstoffdosis (mg/kg) angegeben, die bei 50 % der Tiere eine Senkung der Blutglucosekonzentration um mindestens 25 % im Vergleich zur Kontrollgruppe bewirkt, in Spalte B diejenige, die bei 50 % der Tiere eine Senkung der Blutglucosekonzentration um mindestens 15 % im Vergleich zur Kontrollgruppe bewirkt.

In der Spalte C werden Daten zur akuten Toxizität ($LD_{50}$ Maus p. o.) wiedergegeben.

Tabelle I

| Lfd. Nr. | A<br>$ED_{50}$ (25 %) [mg/kg]<br>Ratte p. o. | B<br>$ED_{50}$ (15 %)[mg/kg]<br>Ratte p. o. | C<br>Toxizität<br>$LD_{50}$ [mg/kg]<br>Maus p. o. |
|---|---|---|---|
| 1 | 194 | >100 | 475 |
| 2 | >343 | >150 | 410* |
| 3 | 54 | 40 | 730 |
| 4 | 41 | 20 | |
| 5 | 24 | 6 | ~300 |
| 6 | 23 | 14 | |
| 7 | 21** | 10 | 230 |
| 8 | 12 | 2 | |
| 9 | 24 | 3 | 275 |
| 10 | 16 | 5 | |
| 11 | | 67 | |
| 12 | | 59 | |

* Zit. Blickens, D. A. ; Riggi, S. J. : Toxicol. Appl. Pharmacol. *14* (1969) 393-400

** $ED_{50}$ (23 %)

Zu Tabelle I :

Spalte A = Dosis, die eine Senkung der Blutglucosekonzentration um 25 % bei 50 % der Tiere bewirkt

Spalte B = Dosis, die eine Senkung der Blutglucosekonzentration um 15 % bei 50 % der Tiere bewirkt

Spalte C = Akute Toxizität ($LD_{50}$ in mg/kg ; Maus p. o.)

In Tabelle II werden Untersuchungen des Einflusses von Vertretern der erfindungsgemäßen Verbindungen auf die Blutglucosekonzentration nüchterner stoffwechselgesunder Meerschweinchen nach einmaliger oraler Substanzgabe von 0,056-0,6 mmol/kg Körpergewicht innerhalb von 5 Stunden wiedergegeben.

In Spalte A ist die Wirkstoffdosis (mg/kg) angegeben, die bei 50 % der Tiere eine Senkung der Blutglucosekonzentration um mindestens 25 % im Vergleich zur Kontrollgruppe bewirkt, in Spalte B diejenige, die bei 50 % der Tiere eine Senkung der Blutglucosekonzentration um mindestens 15 % im Vergleich zur Kontrollgruppe bewirkt.

In der Spalte C werden Daten zur akuten Toxizität ($LD_{50}$ Meerschweinchen p. o.) wiedergegeben.

Tabelle II

| Lfd. Nr. | A<br>$ED_{50}$ (25 %) [mg/kg]<br>Meerschweinchen p. o. | B<br>$ED_{50}$ (15 %) [mg/kg]<br>Meerschweinchen p. o. | C<br>Toxizität<br>$LD_{50}$ [mg/kg]<br>Meerschw. p. o. |
|---|---|---|---|
| 1 | 40-50** | - | 58* |
| 2 | 20-25*** | - | 47* |
| 3 | 22 | 11 | |
| 4 | 26 | 12 | 500-600 |
| 5 | < 24 | 3 | |
| 6 | 11 | 6 | 300-400 |
| 7 | 21 | 11 | |
| 8 | 7 | 3 | 500-600 |
| 9 | < 24 | 5 | |
| 10 | 8 | 3 | 300-400 |

* zit. Proske, G. ; Osterloh, G. ; Beckmann, R. ; Lagler, F. ; Michael, G. ; Mückter, H. : Arzneim. Forsch. *12* (1962) 314-318

** zit. Beckmann, R. : Unveröffentlichte Untersuchungen 1960 in Hdb. exp. Pharmakol. XXIX, S. 477, Springer Berlin 1971.

*** zit. Ungar, G. ; Greedman, L. ; Schapiro, S.L. : Proc. Soc. exp. Biol. (N.Y.) *95* (1957) 190-192.

Zu Tabelle II

Spalte A = Dosis, die eine Senkung der Blutglucosekonzentration um 25 % bei 50 % der Tiere bewirkt

Spalte B = Dosis, die eine Senkung der Blutglucosekonzentration um 15 % bei 50 % der Tiere bewirkt

Spalte C = Akute Toxizität ($LD_{50}$ in mg/kg ; Meerschweinchen p. o., vorläufige Untersuchungen)

Die Ermittlung der pharmakologischen Eigenschaften erfolgte nach folgenden Methoden :

1. Blutglucosebestimmung nach einmaliger oraler Applikation

Zur Verwendung kommen junge männliche Sprague-Dawley-Ratten (Körpergewicht 150-200 g) und junge mischrassige Meerschweinchen (Körpergewicht 250-300 g). Die Haltung der Tiere erfolgt in Makrolon-Käfigen mit bis zu 4 Tieren pro Käfig (Raumtemperatur 23 °C, rel. Luftfeuchtigkeit 55 %, fester Tag/Nacht-Rhythmus (12/12 h), Standarddiät Altromin®). Den Ratten wird 18 Stunden (Meerschweinchen 42 Stunden) vor der 1. Blutabnahme das Futter entzogen. Wasseraufnahme erfolgt ad libitum. Blutentnahmen erfolgen unmittelbar vor und 3 und 5 Stunden nach Substanzgabe durch Punktion aus dem retro-orbitalen Plexus.

Nach Enteiweißung mit Perchlorsäure erfolgt die Blutglucosebestimmung mittels der enzymatischen HK/G-6-PDH-Methode nach R. Richterich (Klinische Chemie, Theorie und Praxis, 3. Aufl. 1971, S. Karger Verlag, Zürich-Basel, Seite 275). Zum Vergleich wird jeweils eine mit reinem Lösungsmittel behandelte Kontrollgruppe mituntersucht.

2. Bestimmung der Toxizität

Die Toxizitätsuntersuchungen werden an weiblichen NMRI-Mäusen (Körpergewicht 22-26 g) durchgeführt. Die Tiere (5 Tiere pro Dosis) erhalten 18 Stunden vor der Behandlung das Futter (Altromin®) auf 50 g/50 Tiere reduziert und Wasser ad libitum. Verschiedene Dosen der Substanzen werden oral mittels Schlundsonde verabreicht (Volumen 10 ml/kg). Die Beobachtungsdauer beträgt 7 Tage. Die $LD_{50}$, d. h. die Dosis, bei der 50 % der Tiere sterben, wird aus der Dosiswirkungskurve graphisch ermittelt.

Es sei darauf hingewiesen, daß die ermittelten Toxizitätswerte vom Nahrungszustand der eingesetzten Tiere abhängig sind. Beispielsweise liegt die $LD_{50}$ p. o. von Ratten, die lediglich 4 Stunden nüchtern gehalten wurden, über 1 g/kg Körpergewicht.

**Ansprüche** (für die Vertragsstaaten : BE, CH, DE, FR, GB, IT, LU, NL, SE)

1. Substituierte Oxirancarbonsäuren der allgemeinen Formel I

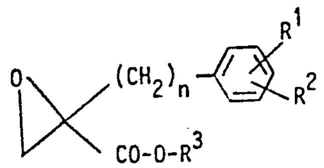

(I)

worin
$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,
$R^2$ eine der Bedeutungen von $R^1$ hat,
$R^3$ ein Wasserstoffatom oder eine $C_1$-$C_4$ Alkylgruppe und
n eine ganze Zahl von 1 bis 8 bedeuten,
sowie die Salze der Carbonsäuren.

2. Substituierte Oxirancarbonsäuren nach Anspruch 1, gekennzeichnet durch die allgemeine Formel I*

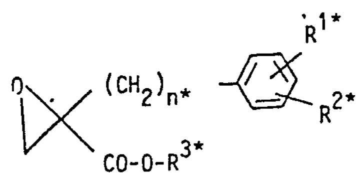

(I*)

worin
$R^{1*}$ und $R^{2*}$ meta- oder para-ständig sind und
$R^{1*}$ ein Wasserstoffatom, ein Chloratom, eine Methylgruppe, eine Methoxygruppe oder eine Trifluormethylgruppe,
$R^{2*}$ ein Wasserstoffatom oder ein Chloratom,
$R^{3*}$ ein Wasserstoffatom oder eine $C_1$-$C_4$ Alkylgruppe und
n* eine ganze Zahl von 3 bis 7 bedeuten,
sowie die Salze der Carbonsäuren.

3. Verbindungen nach Anspruch 2, in denen n* eine ganze Zahl von 3 bis 5 bedeuten.

4. Substituierte Oxirancarbonsäuren nach Anspruch 1, gekennzeichnet durch die allgemeine Formel I**

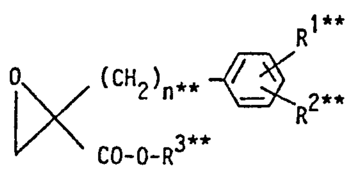

(I**)

worin
$R^{1**}$ und $R^{2**}$ meta- oder para-ständig sind und
$R^{1**}$ ein Wasserstoffatom, ein Chloratom oder eine Trifluormethylgruppe,
$R^{2**}$ ein Wasserstoffatom,
$R^{3**}$ ein Wasserstoffatom, eine Methylgruppe oder eine Ethylgruppe und
n** 3 oder 4 bedeuten,
sowie die pharmakologisch verträglichen Salze der Carbonsäuren mit anorganischen oder organischen Basen.

5. Verbindungen nach Anspruch 4, in denen $R^{1**}$ ein Wasserstoffatom oder ein Chloratom bedeutet und $R^{2**}$, $R^{3**}$ und n** die dort angegebene Bedeutung haben.

6. Substituierte Oxirancarbonsäuren nach Anspruch 1, gekennzeichnet durch die allgemeinen Formel I***

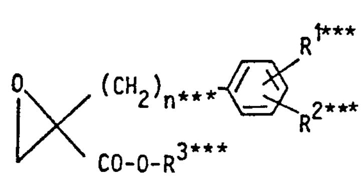

(I***)

worin

$R^{1***}$ und $R^{2***}$ meta- oder para-ständig sind und

$R^{1***}$ ein Wasserstoffatom, ein Chloratom oder eine Trifluormethylgruppe,

$R^{2***}$ ein Wasserstoffatom,

$R^{3***}$ ein Wasserstoffatom, eine Methylgruppe oder eine Ethylgruppe und

$n^{***}$ 5 bedeuten,

sowie die pharmakologisch verträglichen Salze der Carbonsäuren mit anorganischen und organischen Basen.

7. 2-[3-Chlorphenyl)-propyl]-oxiran-2-carbonsäure, ihr Ethylester und ihre pharmakologisch verträglichen Salze mit anorganischen und organischen Basen.

8. 2-[3-(4-Chlorphenyl)-propyl]-oxiran-2-carbonsäure, ihr Ethylester und ihre pharmakologisch verträglichen Salze mit anorganischen und organischen Basen.

9. 2-[3-(3-Trifluormethylphenyl)-propyl]-oxiran-2-carbonsäure, ihr Ethylester und ihre pharmakologisch verträglichen Salze mit anorganischen und organischen Basen.

10. 2-(5-Phenylpentyl)-oxiran-2-carbonsäure, ihr Ethylester und ihre pharmakologisch verträglichen Salze mit anorganischen und organischen Basen.

11. 2-[5-(4-Chlorphenyl)-pentyl]-oxiran-2-carbonsäure, ihr Ethylester und ihre pharmakologisch verträglichen Salze mit anorganischen und organischen Basen.

12. Arzneimittel, enthaltend eine oder mehrere der substituierten Oxirancarbonsäuren der allgemeinen Formel I

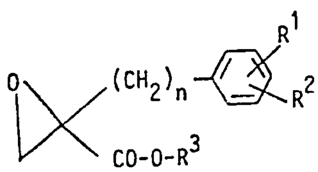

(I)

worin

$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,

$R^2$ eine der Bedeutungen von $R^1$ hat,

$R^3$ ein Wasserstoffatom oder eine $C_1$-$C_4$ Alkylgruppe und

n eine ganze Zahl von 1 bis 8 bedeuten,

und/oder die pharmakologisch verträglichen Salze der Säuren mit anorganischen oder organischen Basen.

13. Arzneimittel nach Anspruch 12, enthaltend eine oder mehrere der substituierten Oxirancarbonsäuren der allgemeinen Formel I*, I** oder I*** und/oder die pharmakologisch verträglichen Salze der Säuren mit anorganischen oder organischen Basen.

14. Verfahren zur Herstellung der substituierten Oxirancarbonsäuren der allgemeinen Formel I

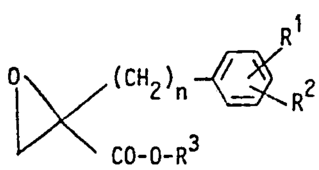

(I)

worin

$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,

$R^2$ eine der Bedeutungen von $R^1$ hat,

$R^3$ ein Wasserstoffatom oder eine $C_1$-$C_4$ Alkylgruppe und

n eine ganze Zahl von 1 bis 8 bedeuten,

sowie der Salze der Säuren, dadurch gekennzeichnet, daß man eine substituierte 2-Methylencarbonsäure der allgemeinen Formel II

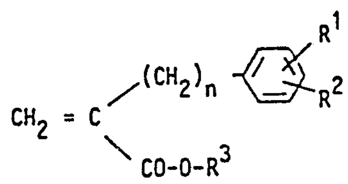

(II)

wobei

$R^1$, $R^2$, $R^3$ und n die vorstehend angegebene Bedeutung haben, oxidiert und gegebenenfalls anschließend die erhaltenen Niederalkylester verseift oder die erhaltenen Säuren in die Salze oder $C_1$-$C_4$ Alkylester überführt.

15. Verfahren nach Anspruch 14, dadurch gekennzeichnet, daß man zur Herstellung der substituierten Oxirancarbonsäuren der allgemeinen Formel I* α-Methylencarbonsäuren der allgemeinen Formel II*

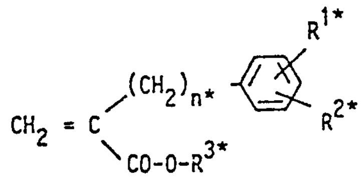

(II*)

worin

$R^{1*}$, $R^{2*}$, $R^{3*}$ und n* die in Anspruch 2 angegebene Bedeutung haben, einsetzt.

16. Verfahren nach Anspruch 15, dadurch gekennzeichnet, daß man Verbindungen II* einsetzt, in denen n* eine ganze Zahl von 3 bis 5 bedeutet.

17. Verfahren nach Anspruch 14, dadurch gekennzeichnet, daß man zur Herstellung der substituierten Oxirancarbonsäuren der allgemeinen Formel I** α-Methylencarbonsäuren der allgemeinen Formel II**

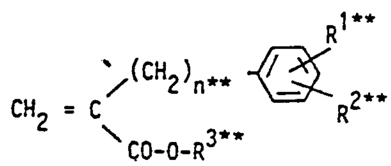

(II**)

worin

$R^{1**}$, $R^{2**}$, $R^{3**}$ und n** die in Anspruch 4 angegebene Bedeutung haben, einsetzt.

18. Verfahren nach Anspruch 17, dadurch gekennzeichnet, daß man Verbindungen II** einsetzt, in denen $R^{1**}$ ein Wasserstoffatom oder ein Chloratom bedeutet.

19. Verfahren nach Anspruch 14, dadurch gekennzeichnet, daß man zur Herstellung der substituierten Oxirancarbonsäuren der allgemeinen Formel I*** α-Methylencarbonsäuren der allgemeinen Formel II***

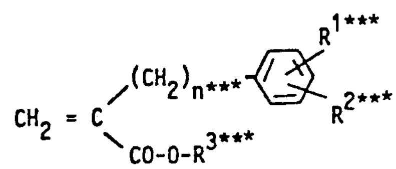

(II***)

worin

$R^{1***}$, $R^{2***}$, $R^{3***}$ und n*** die in Anspruch 6 angegebene Bedeutung haben, einsetzt.

20. Verbindungen nach einem oder mehreren der Ansprüche 1 bis 11 zur Anwendung bei der Bekämpfung von Krankheiten, die auf Störungen des Glucose- und Fettstoffwechsels beruhen.

**Ansprüche** (für den Vertragsstaat AT)

1. Verfahren zur Herstellung der substituierten Oxirancarbonsäuren der allgemeinen Formel I

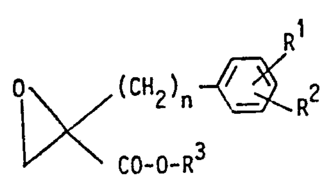

(I)

worin

$R^1$ ein Wasserstoffatom, ein Halogenatom, eine Hydroxygruppe, eine $C_1$-$C_4$ Alkylgruppe, eine $C_1$-$C_4$ Alkoxygruppe oder eine Trifluormethylgruppe bedeutet,

$R^2$ eine der Bedeutungen von $R^1$ hat,

$R^3$ ein Wasserstoffatom oder eine $C_1$-$C_4$ Alkylgruppe und

n eine ganze Zahl von 1 bis 8 bedeuten,

sowie der Salze der Säuren, dadurch gekennzeichnet, daß man eine substituierte α-Methylencarbonsäure der allgemeinen Formel II

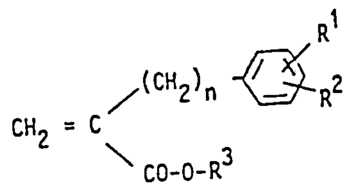

(II)

wobei

$R^1$, $R^2$, $R^3$ und n die vorstehend angegebene Bedeutung haben, oxydiert und gegebenenfalls anschließend die erhaltenen Niederalkylester verseift oder die erhaltenen Säuren in die Salze oder $C_1$-$C_4$ Alkylester überführt.

2. Verfahren nach Anspruch 1, dadurch gekennzeichnet, daß man zur Herstellung der substituierten Oxirancarbonsäuren der allgemeinen Formel I*

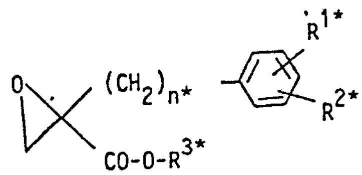

(I*)

worin

$R^{1*}$ und $R^{2*}$ meta- oder para-ständig sind und

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom,

$R^{3*}$ ein Wasserstoffatom oder eine $C_1$-$C_4$ Alkylgruppe und

n* eine ganze Zahl von 3 bis 7 bedeuten,

α-Methylencarbonsäuren der allgemeinen Formel II*

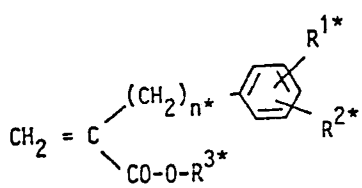

(II*)

worin

$R^{1*}$, $R^{2*}$, $R^{3*}$ und n* die oben angegebene Bedeutung haben, einsetzt.

3. Verfahren nach Anspruch 2, dadurch gekennzeichnet, daß man Verbindungen II* einsetzt, in denen n eine ganze Zahl von 3 bis 5 bedeutet.

4. Verfahren nach Anspruch 1, dadurch gekennzeichnet, daß man zur Herstellung der substituierten Oxirancarbonsäuren der allgemeinen Formel I**

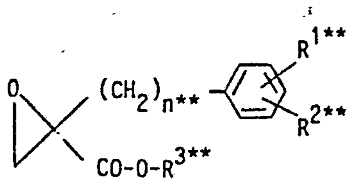

(I**)

worin
$R^{1**}$ und $R^{2**}$ meta- oder para-ständig sind und
$R^{1**}$ ein Wasserstoffatom, ein Chloratom oder eine Trifluormethylgruppe,
$R^{2**}$ ein Wasserstoffatom,
$R^{3**}$ ein Wasserstoffatom, eine Methylgruppe oder eine Ethylgruppe und
$n^{**}$ 3 oder 4 bedeuten,
α-Methylencarbonsäuren der allgemeinen Formel II**

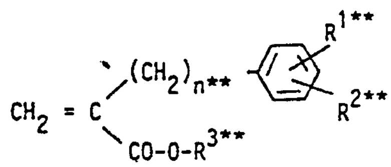

(II**)

worin
$R^{1**}$, $R^{2**}$, $R^{3**}$ und $n^{**}$ die oben angegebene Bedeutung haben, einsetzt.

5. Verfahren nach Anspruch 4, dadurch gekennzeichnet, daß man Verbindungen II** einsetzt, in denen $R^{1**}$ ein Wasserstoffatom oder ein Chloratom bedeutet.

6. Verfahren nach Anspruch 1, dadurch gekennzeichnet, daß man zur Herstellung der substituierten Oxirancarbonsäuren der allgemeinen Formel I***

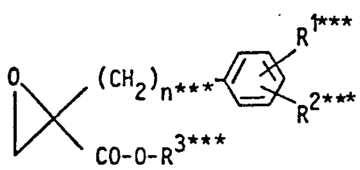

(I***)

worin
$R^{1***}$ und $R^{2***}$ meta- oder para-ständig sind und
$R^{1***}$ ein Wasserstoffatom, ein Chloratom oder eine Trifluormethylgruppe,
$R^{2***}$ ein Wasserstoffatom,
$R^{3***}$ ein Wasserstoffatom, eine Methylgruppe oder eine Ethylgruppe und
$n^{***}$ 5 bedeuten,
α-Methylencarbonsäuren der allgemeinen Formel II***

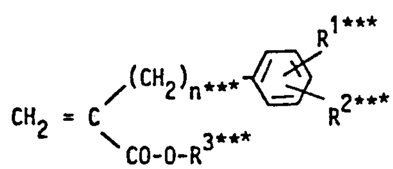

(II***)

worin
$R^{1***}$, $R^{2***}$, $R^{3***}$ und $n^{***}$ die oben angegebene Bedeutung haben, einsetzt.

7. Verfahren nach Anspruch 5, dadurch gekennzeichnet, daß man 2-[3-(3-Chlorphenyl)-propyl]-oxiran-2-carbonsäure, ihre Ethylester und ihre pharmakologisch verträglichen Salze mit anorganischen und organischen Basen herstellt.

8. Verfahren nach Anspruch 5, dadurch gekennzeichnet, daß man 2-[3-(4-Chlorphenyl)-propyl]-oxiran-2-carbonsäure, ihre Ethylester und ihre pharmakologisch verträglichen Salze mit anorganischen und organischen Basen herstellt.

9. Verfahren nach Anspruch 4, dadurch gekennzeichnet, daß man 2-[3-(3-Trifluormethylphenyl)-propyl]-oxiran-2-carbonsäure, ihre Ethylester und ihre pharmakologisch verträglichen Salze mit anorganischen und organischen Basen herstellt.

10. Verfahren nach Anspruch 6, dadurch gekennzeichnet, daß man 2-(5-Phenylpentyl)-oxiran-2-carbonsäure, ihre Ethylester und ihre pharmakologisch verträglichen Salze mit anorganischen und organischen Basen herstellt.

11. Verfahren nach Anspruch 6, dadurch gekennzeichnet, daß man 2-[5-(4-Chlorphenyl)-pentyl]-oxiran-2-carbonsäure, ihre Ethylester und ihre pharmakologisch verträglichen Salze mit anorganischen und organischen Basen herstellt.

**Claims** (for the Contracting States : BE, CH, DE, FR, GB, IT, LU, NL, SE)

1. Substituted oxiranecarboxylic acids of the general formula I

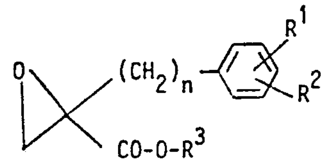

(I)

wherein

$R^1$ denotes a hydrogen atom, a halogen atom, a hydroxyl group, a (C1-C4) alkyl group, a (C1-C4) alkoxy group or a trifluoromethyl group,

$R^2$ has one of the meanings of $R^1$,

$R^3$ denotes a hydrogen atom or a (C1-C4) alkyl group and

n denotes an integer from 1 to 8,

and the salts of the carboxylic acids.

2. Substituted oxiranecarboxylic acids according to Claim 1, characterised by the general formula I*

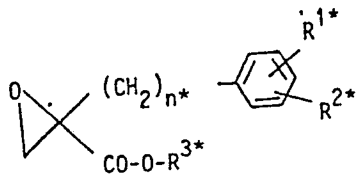

(I*)

wherein

$R^{1*}$ and $R^{2*}$ are in the meta-position or para-position and

$R^{1*}$ denotes a hydrogen atom, a chlorine atom, a methyl group, a methoxy group or a trifluoromethyl group,

$R^{2*}$ denotes a hydrogen atom or a chlorine atom,

$R^{3*}$ denotes a hydrogen atom or a (C1-C4) alkyl group and

n* denotes an integer from 3 to 7

and the salts of the carboxylic acids.

3. Compounds according to Claim 2, wherein n* denotes an integer from 3 to 5.

4. Substituted oxiranecarboxylic acids according to Claim 1, characterised by the general formula I**

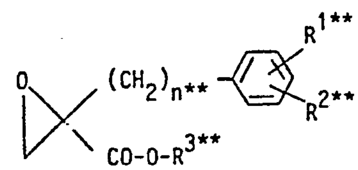

(I**)

wherein

$R^{1**}$ and $R^{2**}$ are in the meta-position or para-position and

$R^{1**}$ denotes a hydrogen atom, a chlorine atom or a trifluoromethyl group,

$R^{2**}$ denotes a hydrogen atom,

$R^{3**}$ denotes a hydrogen atom, a methyl group or an ethyl group and

n** denotes 3 or 4,

and the pharmacologically acceptable salts of the carboxylic acids with inorganic or organic bases.

5. Compounds according to Claim 4, wherein $R^{1**}$ denotes a hydrogen atom or a chlorine atom and $R^{2**}$, $R^{3**}$ and n** have the meaning indicated in Claim 4.

6. Substituted oxiranecarboxylic acids according to Claim 1, characterised by the general formula I***,

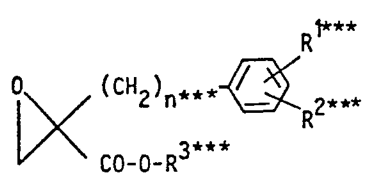

(I***)

wherein

$R^{1***}$ and $R^{2***}$ are in the meta-position or para-position and

$R^{1***}$ denotes a hydrogen atom, a chlorine atom or a trifluoromethyl group,

$R^{2***}$ denotes a hydrogen atom,

$R^{3***}$ denotes a hydrogen atom, a methyl group or an ethyl group and

$n^{***}$ denotes 5,

and the pharmacologically acceptable salts of the carboxylic acids with inorganic or organic bases.

7. 2-[3-(3-Chlorophenyl)-propyl]-oxirane-2-carboxylic acid, its ethyl ester and pharmacologically acceptable salts with inorganic and organic bases.

8. 2-[3-(4-Chlorophenyl)-propyl]-oxirane-2-carboxylic acid, its ethyl ester and pharmacologically acceptable salts with inorganic and organic bases.

9. 2-[3-(3-Trifluoromethylphenyl)-propyl]-oxirane-2-carboxylic acid, its ethyl ester and pharmacologically acceptable salts with inorganic and organic bases.

10. 2-(5-Phenylpentyl)-oxirane-2-carboxylic acid, its ethyl ester and pharmacologically acceptable salts with inorganic and organic bases.

11. 2-[5-(4-Chlorophenyl)-pentyl]-oxirane-2-carboxylic acid, its ethyl ester and pharmacologically acceptable salts with inorganic and organic bases.

12. Medicaments containing one or more of the substituted oxiranecarboxylic acids of the general formula I

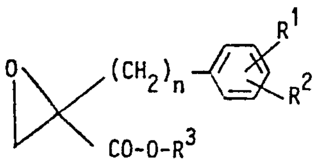

(I)

wherein

$R^1$ denotes a hydrogen atom, a halogen atom, a hydroxyl group, a (C1-C4) alkyl group, a (C1-C4) alkoxy group or a trifluoromethyl group,

$R^2$ has one of the meanings of $R^1$,

$R^3$ denotes a hydrogen atom or a (C1-C4) alkyl group and

n denotes an integer from 1 to 8,

and/or the pharmacologically acceptable salts of the acids with inorganic or organic bases.

13. Medicaments according to Claim 12 containing one or more of the substituted oxiranecarboxylic acids of the general formula I*, I** or I*** and/or the pharmacologically acceptable salts of the acids with inorganic or organic bases.

14. Process for the preparation of the substituted oxiranecarboxylic acids of the general formula I

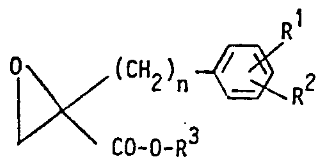

(I)

wherein

$R^1$ denotes a hydrogen atom, a halogen atom, a hydroxyl group, a (C1-C4) alkyl group, a (C1-C4) alkoxy group or a trifluoromethyl group,

$R^2$ has one of the meanings of $R^1$,

$R^3$ denotes a hydrogen atom or a (C1-C4) alkyl group and

n denotes an integer from 1 to 8,

and the salts of the acids, characterised in that substituted α-methylenecarboxylic acids of the general formula II

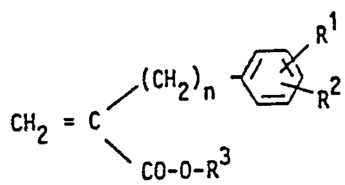

(II)

wherein

$R^1$, $R^2$, $R^3$ and n have the meaning indicated above, are oxidised and the resulting lower alkyl esters

are then optionally saponified or the resulting acids are then optionally converted into the salts or (C1-C4) alkyl esters.

15. Process according to Claim 14, characterised in that, for the preparation of the substituted oxiranecarboxylic acids of the general formula I*, α-methylenecarboxylic acids of the general formula II*

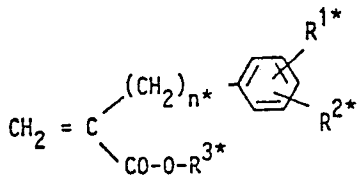

(II*)

wherein

$R^{1*}$, $R^{2*}$, $R^{3*}$ and n* have the meaning indicated in Claim 2, are employed.

16. Process according to Claim 15, characterised in that compounds II*, wherein n* is an integer from 3 to 5, are employed.

17. Process according to Claim 14, characterised in that, for the preparation of the substituted oxiranecarboxylic acids of the general formula I**, α-methylenecarboxylic acids of the general formula II**

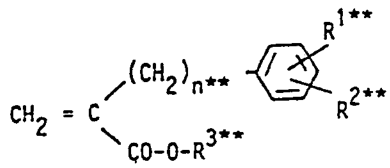

(II**)

wherein

$R^{1**}$, $R^{2**}$, $R^{3**}$ and n** have the meaning indicated in Claim 4, are employed.

18. Process according to Claim 17, characterised in that compounds II**, wherein $R^{1**}$ denotes a hydrogen atom or chlorine atom, are employed.

19. Process according to Claim 14, characterised in that, for the preparation of the substituted oxiranecarboxylic acids of the general formula I***, α-methylenecarboxylic acids of the general formula II***

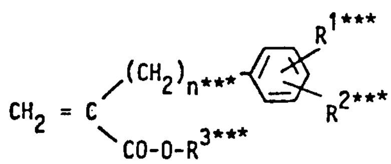

(II***)

wherein

$R^{1***}$, $R^{2***}$, $R^{3***}$ and n*** have the meaning indicated in Claim 6, are employed.

20. Compounds according to one or more of Claims 1 to 11 for use in combating illnesses based on disorders in the metabolism of glucose and fat.

**Claims** (for the Contracting State AT)

1. Process for the preparation of the substituted oxiranecarboxylic acids of the general formula I

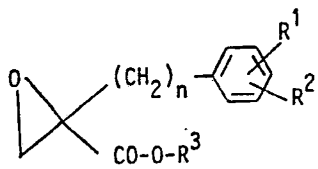

(I)

wherein

$R^1$ denotes a hydrogen atom, a halogen atom, a hydroxyl group, a (C1-C4) alkyl group, a (C1-C4) alkoxy group or a trifluoromethyl group,

$R^2$ has one of the meanings of $R^1$,

$R^3$ denotes a hydrogen atom or a (C1-C4) alkyl group and

n denotes an integer from 1 to 8,

and the salts of the acids, characterised in that substituted α-methylenecarboxylic acids of the general formula II

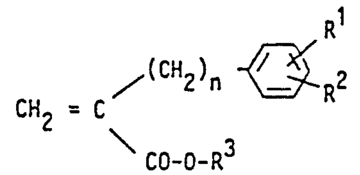

(II)

wherein

$R^1$, $R^2$, $R^3$ and n have the meaning indicated above, are oxidised and the resulting lower alkyl esters are then optionally saponified or the resulting acids are then optionally converted into the salts or (C1-C4) alkyl esters.

2. Process according to Claim 1, characterised in that, for the preparation of the substituted oxiranecarboxylic acids of the general formula I*

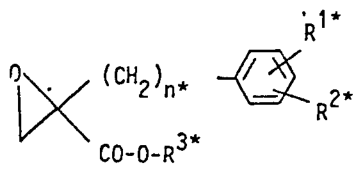

(I*)

wherein

$R^{1*}$ and $R^{2*}$ are in the meta-position or para-position and

$R^{1*}$ denotes a hydrogen atom, a chlorine atom, a methyl group, a methoxy group or a trifluoromethyl group,

$R^{2*}$ denotes a hydrogen atom or a chlorine atom,

$R^{3*}$ denotes a hydrogen atom or a (C1-C4) alkyl group and

n* denotes an integer from 3 to 7,

α-methylenecarboxylic acids of the general formula II*

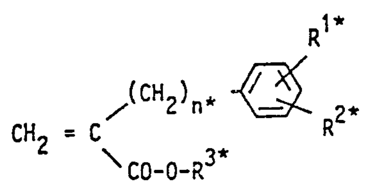

(II*)

wherein

$R^{1*}$, $R^{2*}$, $R^{3*}$ and n* have the meaning indicated above, are employed.

3. Process according to Claim 2, characterised in that compounds II*, wherein n* is an integer from 3 to 5, are employed.

4. Process according to Claim 1, characterised in that, for the preparation of the substituted oxiranecarboxylic acids of the general formula I**

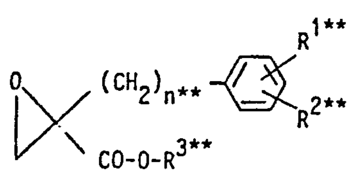

(I**)

wherein

$R^{1**}$ and $R^{2**}$ are in the meta-position or para-position and and

$R^{1**}$ denotes a hydrogen atom, a chlorine atom or a trifluoromethyl group,

$R^{2**}$ denotes a hydrogen atom,

$R^{3**}$ denotes a hydrogen atom, a methyl group or an ethyl group and

n** denotes 3 or 4,

α-methylenecarboxylic acids of the general formula II**

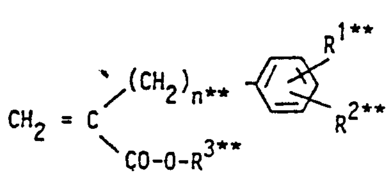

(II**)

wherein

$R^{1**}$, $R^{2**}$, $R^{3**}$ and $n^{**}$ have the meaning indicated above, are employed.

5. Process according to Claim 4, characterised in that compounds $II^{**}$, wherein $R^{1**}$ denotes a hydrogen atom or chlorine atom, are employed.

6. Process according to Claim 1, characterised in that, for the preparation of the substituted oxiranecarboxylic acids of the general formula $I^{***}$

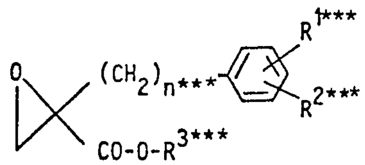

($I^{***}$)

wherein

$R^{1***}$ and $R^{2***}$ are in the meta-position or para-position,

$R^{1***}$ denotes a hydrogen atom, a chlorine atom or a trifluoromethyl group,

$R^{2***}$ denotes a hydrogen atom,

$R^{3***}$ denotes a hydrogen atom, a methyl group or an ethyl group and

$n^{***}$ denotes 5,

α-methylenecarboxylic acids of the general formula $II^{***}$

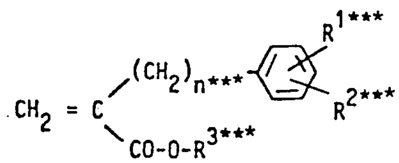

($II^{***}$)

wherein

$R^{1***}$, $R^{2***}$, $R^{3***}$ and $n^{***}$ have the meaning indicated above, are employed.

7. Process according to Claim 5, characterised in that 2-[3-(3-chlorophenyl)-propyl]-oxirane-2-carboxylic acid, its ethyl ester and its pharmacologically acceptable salts with inorganic and organic bases are prepared.

8. Process according to Claim 5, characterised in that 2-[3-(4-chlorophenyl)-propyl]-oxirane-2-carboxylic acid, its ethyl ester and its pharmacologically acceptable salts with inorganic and organic bases are prepared.

9. Process according to Claim 4, characterised in that 2-[3-(3-trifluoromethylphenyl)-propyl]-oxirane-2-carboxylic acid, its ethyl ester and its pharmacologically acceptable salts with inorganic and organic bases are prepared.

10. Process according to Claim 6, characterised in that 2-(5-phenylpentyl)-oxirane-2-carboxylic acid, its ethyl ester and its pharmacologically acceptable salts with inorganic and organic bases are prepared.

11. Process according to Claim 6, characterised in that 2-[5-(4-chlorophenyl)-pentyl]-oxirane-2-carboxylic acid, its ethyl ester and its pharmacologically acceptable salts with inorganic and organic bases are prepared.

**Revendications** (pour les Etats contractants : BE, CH, DE, FR, GB, IT, LU, NL, SE)

1. Acides oxyranecarboxyliques substitués, caractérisés en ce qu'ils répondent à la formule générale I ci-après

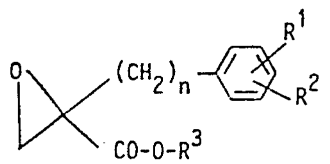

(I)

dans laquelle

$R^1$ représente un atome d'hydrogène, un atome d'halogène, un groupe hydroxy-, un groupe alkyle en $C_1$ à $C_4$, un groupe alcoxy- en $C_1$ à $C_4$ ou un groupe trifluorométhyle,

$R^2$ a une des significations de $R^1$,

$R^3$ représente un atome d'hydrogène ou un groupe alkyle en $C_1$ à $C_4$, et

n est un nombre entier compris entre 1 et 8

ainsi que les sels de ces acides carboxyliques.

2. Acides oxyranecarboxyliques substitués selon la revendication 1, caractérisés en ce qu'ils répondent à la formule générale I* ci-après

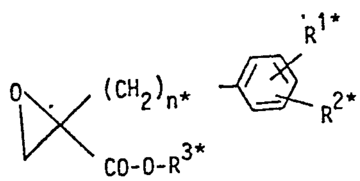

(I*)

dans laquelle

$R^{1*}$ et $R^{2*}$ sont en position méta- ou para- et

$R^{1*}$ représente un atome d'hydrogène, un atome de chlore, un groupe méthyle, un groupe méthoxy- ou un groupe trifluorométhyle,

$R^{2*}$ est un atome d'hydrogène ou de chlore,

$R^{3*}$ est un atome d'hydrogène ou un groupe alkyle en $C_1$ à $C_4$ et,

n* est un nombre entier compris entre 3 et 7,

ainsi que les sels de ces acides carboxyliques.

3. Composés selon la revendication 2, caractérisés en ce que n* représente un nombre entier compris entre 3 et 5.

4. Acides oxyranecarboxyliques substitués selon la revendication 1, caractérisés en ce qu'ils répondent à la formule générale I** ci-après

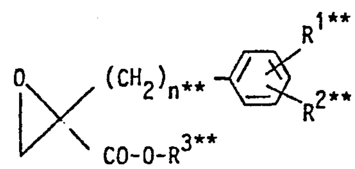

(I**)

dans laquelle

$R^{1**}$ et $R^{2**}$ sont en position méta- ou para- et

$R^{1**}$ représente un atome d'hydrogène, un atome de chlre ou un groupe trifluorométhyle,

$R^{2**}$ est un atome d'hydrogène,

$R^{3**}$ est un atome d'hydrogène, un groupe méthyle ou un groupe éthyle, et

n** est égal à 3 ou 4,

ainsi que les sels pharmacologiquement compatibles de ces acides carboxyliques avec des bases minérales ou organiques.

5. Composés selon la revendication 4, caractérisés en ce que

$R^{1**}$ représente un atome d'hydrogène ou un atome de chlore et

$R^{2**}$, $R^{3**}$ et n** ont la signification mentionnée dans la revendication 4.

6. Acides oxyranecarboxyliques substitués selon la revendication 1, caractérisés en ce qu'ils répondent à la formule générale I*** ci-après :

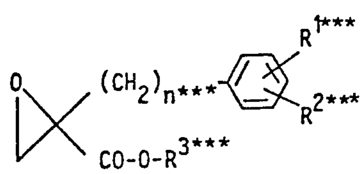

(I***)

dans laquelle

$R^{1***}$ et $R^{2***}$ sont en position méta- ou para- et

$R^{1***}$ représente un atome d'hydrogène, un atome de chlore ou un groupe trifluorométhyle,

$R^{2***}$ est un atome d'hydrogène,

$R^{3***}$ est un atome d'hydrogène, un groupe méthyle ou un groupe éthyle, et

n*** est égal à 5,

ainsi que les sels pharmacologiquement compatibles de ces acides carboxyliques avec des bases

minérales et organiques.

7. L'acide 2-[3-(3-chlorophényl)-propyl]-oxyrane-2-carboxylique, son éthylester et ses sels pharmacologiquement compatibles avec des bases minérales et organiques.

8. L'acide 2-[3-(4-chlorophényl)-propyl]-oxyrane-2-carboxylique, son éthylester et ses sels pharmacologiquement compatibles avec des bases minérales et organiques.

9. L'acide 2-[3-(3-trifluorométhylphényl)-propyl]-oxyrane-2-carboxylique, son éthylester et ses sels pharmacologiquement compatibles avec des bases minérales et organiques.

10. L'acide 2-(5-phénylpentyl)-oxyrane-2-carboxylique, son éthylester et ses sels pharmacologiquement compatibles avec des bases minérales et organiques.

11. L'acide 2-[5-(4-chlorophényl)-pentyl]-oxyrane-2-carboxylique, son éthylester et ses sels pharmacologiquement compatibles avec des bases minérales et organiques.

12. Médicament caractérisé en ce qu'il contient un ou plusieurs des acides oxyranecarboxyliques substitués de formule générale I ci-après

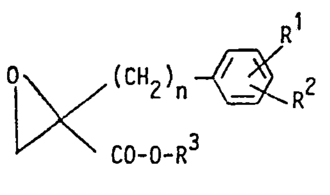

(I)

dans laquelle

$R^1$ représente un atome d'hydrogène, un atome d'halogène, un groupe hydroxy-, un groupe alkyle en $C_1$ à $C_4$, un groupe alcoxy- en $C_1$ à $C_4$ ou un groupe trifluorométhyle,

$R^2$ a une des significations de $R^1$,

$R^3$ est un atome d'hydrogène ou un groupe alkyle en $C_1$ à $C_4$, et

n est un nombre entier compris entre 1 et 8

et/ou les sels pharmacologiquement compatibles de ces acides avec des bases minérales ou organiques.

13. Médicament selon la revendication 12, caractérisé en ce qu'il contient un ou plusieurs des acides oxyranecarboxyliques substitués de formule générale I*, I** ou I*** et/ou les sels pharmacologiquement compatibles de ces acides avec des bases minérales ou organiques.

14. Procédé de préparation des acides oxyranecarboxyliques de formule générale I ci-après

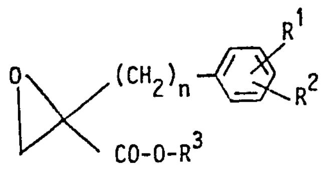

(I)

dans laquelle

$R^1$ représente un atome d'hydrogène, un atome d'halogène, un groupe hydroxy-, un groupe alkyle en $C_1$ à $C_4$, un groupe alcoxy- en $C_1$ à $C_4$ ou un groupe trifluorométhyle,

$R^2$ a une des significations de $R^1$,

$R^3$ représente un atome d'hydrogène ou un groupe alkyle en $C_1$ à $C_4$, et

n est un nombre entier compris entre 1 et 8,

ainsi que les sels de ces acides, caractérisé en ce que l'on oxyde un acide 2-méthylènecarboxylique substitué de formule générale II ci-après

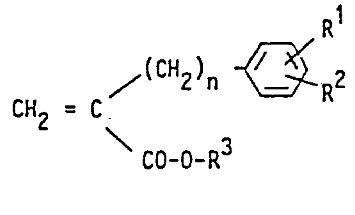

(II)

dans laquelle

$R^1$, $R^2$, $R^3$ et n ont la signification précédemment indiquée, et en ce que l'on saponifie éventuellement ensuite l'alkyl(inférieur)-ester obtenu ou bien on transforme les acides obtenus en les sels ou en alkylesters où l'alkyle est en $C_1$ à $C_4$.

15. Procédé selon la revendication 14, caractérisé en ce que l'on met en œuvre pour la préparation des acides oxyranecarboxyliques substitués de formule générale I*, des acides α-méthylènecarboxyliques de formule générale $II^{*}$ ci-après

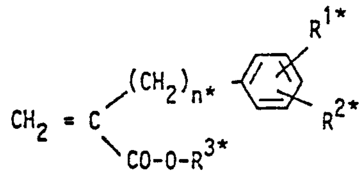

($II^{*}$)

dans laquelle

$R^{1*}$, $R^{2*}$, $R^{3*}$ et $n^{*}$ ont la signification donnée dans la revendication 2.

16. Procédé selon la revendication 15, caractérisé en ce que l'on met en œuvre des composés de formule générale $II^{*}$ dans lesquels $n^{*}$ est un nombre entier compris entre 3 et 5.

17. Procédé selon la revendication 14, caractérisé en ce que l'on met en œuvre pour la préparation des acides oxyranecarboxyliques substitués de formule générale $I^{**}$, des acides α-méthylènecarboxyliques de formule générale $II^{**}$ ci-après

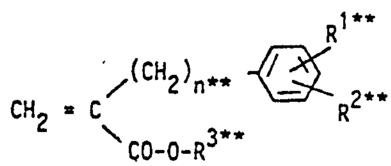

($II^{**}$)

dans laquelle

$R^{1**}$, $R^{2**}$, $R^{3**}$ et $n^{**}$ ont la signification donnée dans la revendication 4.

18. Procédé selon la revendication 17, caractérisé en ce que l'on met en œuvre des composés de formule $II^{**}$, dans lesquels $R^{1**}$ représente un atome d'hydrogène ou un atome de chlore.

19. Procédé selon la revendication 14, caractérisé en ce que l'on met en œuvre pour la préparation des acides oxyranecarboxyliques substitués de formule générale $I^{***}$, des acides α-méthylènecarboxyliques de formule générale $II^{***}$ ci-après

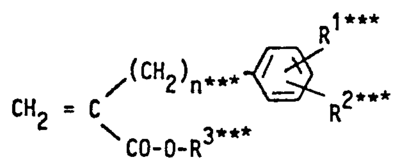

($II^{***}$)

dans laquelle

$R^{1***}$, $R^{2***}$, $R^{3***}$ et $n^{***}$ ont la signification donnée dans la revendication 6.

20. Composés selon une ou plusieurs des revendications 1 à 11, destinés à être utilisés pour le traitement de maladies liées à des perturbations du métabolisme glucidique et lipidique.

**Revendications** (pour l'Etat contractant AT)

1. Procédé de préparation des acides oxyranecarboxyliques de formule générale I ci-après

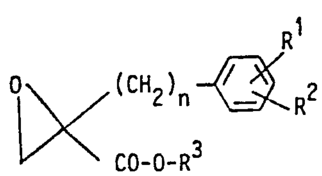

(I)

dans laquelle

$R^1$ représente un atome d'hydrogène, un atome d'halogène, un groupe hydroxy-, un groupe alkyle en $C_1$ à $C_4$, un groupe alcoxy- en $C_1$ à $C_4$ ou un groupe trifluorométhyle,

$R^2$ a une des significations de $R^1$,

$R^3$ représente un atome d'hydrogène ou un groupe alkyle en $C_1$ à $C_4$, et

n est un nombre entier compris entre 1 et 8,

ainsi que les sels de ces acides, caractérisé en ce que l'on oxyde un acide 2-méthylènecarboxylique

substitué de formule générale II ci-après

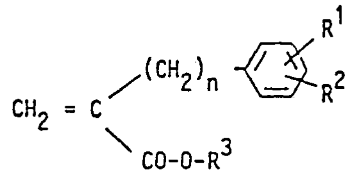

. (II)

dans laquelle

$R^1$, $R^2$, $R^3$ et n ont la signification précédemment indiquée, et en ce que l'on saponifie éventuellement ensuite l'alkyl(inférieur)-ester obtenu ou bien on transforme les acides obtenus en les sels ou en alkylesters où l'alkyle est en $C_1$ à $C_4$.

2. Procédé selon la revendication 1, caractérisé en ce que l'on met en œuvre pour la préparation des acides oxyranecarboxyliques substitués de formule générale I* ci-après :

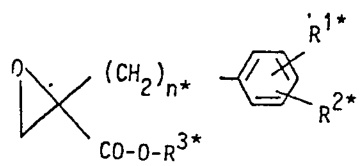

. (I*)

dans laquelle

$R^{1*}$ et $R^{2*}$ sont en position méta- ou para- et

$R^{1*}$ représente un atome d'hydrogène, un atome de chlore, un groupe méthyle, un groupe méthoxy- ou un groupe trifluorométhyle,

$R^{2*}$ est un atome d'hydrogène ou de chlore,

$R^{3*}$ est un atome d'hydrogène ou un groupe alkyle en $C_1$ à $C_4$ et,

n* est un nombre entier compris entre 3 et 7,

des acides α-méthylènecarboxyliques de formule générale II* ci-après

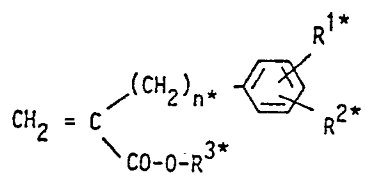

(II*)

dans laquelle

$R^{1*}$, $R^{2*}$, $R^{3*}$ et n* ont la signification donnée ci-dessus.

3. Procédé selon la revendication 2, caractérisé en ce que l'on met en œuvre des composés de formule générale II* dans lesquels n* est un nombre entier compris entre 3 et 5.

4. Procédé selon la revendication 1, caractérisé en ce que l'on met en œuvre pour la préparation des acides oxyranecarboxyliques substitués de formule générale I** ci-après

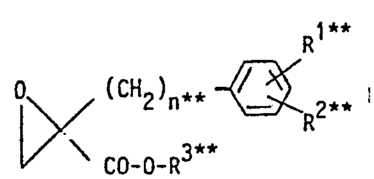

(I**)

dans laquelle

$R^{1**}$ et $R^{2**}$ sont en position méta- ou para- et

$R^{1**}$ représente un atome d'hydrogène, un atome de chlore ou un groupe trifluorométhyle,

$R^{2**}$ est un atome d'hydrogène,

$R^{3**}$ est un atome d'hydrogène, un groupe méthyle ou un groupe éthyle, et

n** est égal à 3 ou 4,

des acides α-méthylènecarboxyliques de formule générale II** ci-après

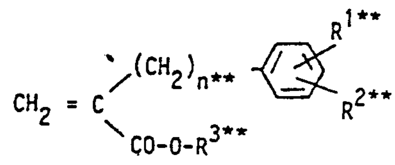

(II**)

dans laquelle

$R^{1**}$, $R^{2**}$, $R^{3**}$ et n** ont la signification donnée ci-dessus.

5. Procédé selon la revendication 4, caractérisé en ce que l'on met en œuvre des composés de formule II**, dans lesquels $R^{1**}$ représente un atome d'hydrogène ou un atome de chlore.

6. Procédé selon la revendication 1, caractérisé en ce que l'on met en œuvre pour la préparation des acides oxyranecarboxyliques substitués de formule générale I*** ci-après

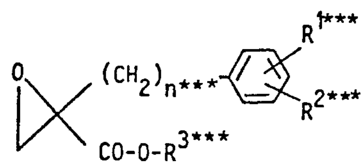

(I***)

dans laquelle

$R^{1***}$ et $R^{2***}$ sont en position méta- ou para- et

$R^{1***}$ représente un atome d'hydrogène, un atome de chlore ou un groupe trifluorométhyle,

$R^{2***}$ est un atome d'hydrogène,

$R^{3***}$ est un atome d'hydrogène, un groupe méthyle ou un groupe éthyle, et

n*** est égal à 5,

des acides α-méthylènecarboxyliques de formule générale II*** ci-après

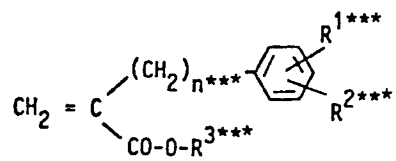

(II***)

dans laquelle

$R^{1***}$, $R^{2***}$, $R^{3***}$ et n*** ont la signification donnée ci-dessus.

7. Procédé selon la Revendication 5, caractérisé en ce qu'on prépare l'acide 2-[3-(3-chlorophényl)-propyl]-oxyrane-2-carboxylique, son éthylester et ses sels pharmacologiquement compatibles avec des bases minérales et organiques.

8. Procédé selon la Revendication 5, caractérisé en ce qu'on prépare l'acide 2-[3-(4-chlorophényl)-propyl]-oxyrane-2-carboxylique, son éthylester et ses sels pharmacologiquement compatibles avec des bases minérales et organiques.

9. Procédé selon la Revendication 4, caractérisé en ce qu'on prépare l'acide 2-[3-(3-trifluorométhyl-phényl)-propyl]-oxyrane-2-carboxylique, son éthylester et ses sels pharmacologiquement compatibles avec des bases minérales et organiques.

10. Procédé selon la Revendication 6, caractérisé en ce qu'on prépare l'acide 2-(5-phénylpentyl)-oxyrane-2-carboxylique, son éthylester et ses sels pharmacologiquement compatibles avec des bases minérales et organiques.

11. Procédé selon la Revendication 6, caractérisé en ce qu'on prépare l'acide 2-[5-(4-chlorophényl)-pentyl]-oxyrane-2-carboxylique, son éthylester et ses sels pharmacologiquement compatibles avec des bases minérales et organiques.